(19) Europäisches Patentamt / European Patent Office / Office européen des brevets

(11) **EP 3 933 023 A1**

(12) **EUROPEAN PATENT APPLICATION**
published in accordance with Art. 153(4) EPC

(43) Date of publication:
**05.01.2022 Bulletin 2022/01**

(51) Int Cl.:
*C12M 1/12* *(2006.01)*      *B01D 61/14* *(2006.01)*
*B01D 69/02* *(2006.01)*      *B01D 69/12* *(2006.01)*
*B01D 71/26* *(2006.01)*      *B01D 71/38* *(2006.01)*
*C12N 7/02* *(2006.01)*      *C12Q 1/04* *(2006.01)*
*G01N 33/48* *(2006.01)*

(21) Application number: 20770985.8

(22) Date of filing: 28.01.2020

(86) International application number:
**PCT/JP2020/002968**

(87) International publication number:
**WO 2020/183955 (17.09.2020 Gazette 2020/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 14.03.2019 JP 2019047541
14.03.2019 JP 2019047540

(71) Applicants:
• **Teijin Limited**
**Osaka 530-0005 (JP)**
• **Visgene, Ltd**
**Minoh-shi, Osaka 562-0033 (JP)**

(72) Inventors:
• **NAMBU, Mami**
**Osaka-shi, Osaka 530-0005 (JP)**
• **IKUTA, Yoshikazu**
**Osaka-shi, Osaka 530-0005 (JP)**
• **NAGAO, Yu**
**Osaka-shi, Osaka 530-0005 (JP)**
• **KAIHATSU, Kunihiro**
**Ibaraki-shi, Osaka 567-0047 (JP)**
• **YUMEN, Ikuko**
**Ibaraki-shi, Osaka 567-0047 (JP)**
• **OGATA, Katsuya**
**Ibaraki-shi, Osaka 567-0047 (JP)**
• **AOKI, Daisuke**
**Ibaraki-shi, Osaka 567-0047 (JP)**

(74) Representative: **Carpmaels & Ransford LLP**
**One Southampton Row**
**London WC1B 5HA (GB)**

(54) **MEMBRANE FOR CONCENTRATING BIOLOGICAL PARTICLES, CONCENTRATING DEVICE, CONCENTRATING SYSTEM, CONCENTRATING METHOD, AND METHOD FOR DETECTING BIOLOGICAL PARTICLES**

(57) A concentration membrane for use in concentrating biological particles, including: a hydrophilic composite porous membrane including: a porous substrate; and a hydrophilic resin with which at least one main surface and inner surfaces of pores of the porous substrate are coated, the hydrophilic composite porous membrane having a ratio t/x of a membrane thickness t (μm) to an average pore diameter x (μm), as measured with a perm porometer, of from 50 to 630. A concentration device 10 for biological particles 50 including: a housing 20 having an inlet 21 and an outlet 22, in which, due to a differential pressure between the inlet 21 and the outlet 22, a liquid to be treated 40 containing biological particles 50 and water is injected from the inlet 21 and discharged from the outlet 22; a concentration membrane 30 provided to separate the inlet 21 and the outlet 22 from each other in the housing 20, the concentration membrane 30 being a hydrophilic porous membrane onto which the biological particles 50 are not adsorbed, the concentration membrane 30 allowing an effluent 42, which is a liquid having a concentration that is a concentration of the biological particles 50 subtracted from a concentration of the liquid to be treated 40, to permeate from a surface on a side of the inlet 21 to a surface on a side of the outlet 22; and a concentration space portion 24 which is a space on an upstream side of the concentration membrane 30 in the housing 20 and stores a concentrated liquid 41 which is a liquid having a concentration that is a concentration of the biological particles 50 added to a concentration of the liquid to be treated 40 by the concentration membrane 30.

EP 3 933 023 A1

# FIG.4

**Description**

Technical Field

The present invention relates to a concentration membrane, a concentration device, a concentration system, and a concentration method for biological particles, and a method for detecting biological particles.

Background Art

Patent Document 1 discloses that a bundle of polyethylene porous hollow fiber membranes having a surface coated with an ethylene/vinyl alcohol copolymer is used for capturing microorganisms.
Patent Document 2 discloses that a filter membrane having a bubble point pore diameter not exceeding 1.0 $\mu$m is used for capturing microorganisms.
Patent Document 3 discloses a method for producing a hydrophilized polymer microporous membrane in which a hydrophilic monomer is radiation-grafted onto the surface of a polymer microporous membrane made of a hydrophobic resin.
Patent Document 4 discloses a hydrophilic microporous membrane obtained by copolymerizing a hydrophilic monomer having one vinyl group and a crosslinking agent having two or more vinyl groups with a polymer microporous membrane by a graft polymerization method.
Patent Document 5 discloses that a porous hollow fiber membrane in which a porous hollow fiber substrate of a polyolefin is coated with a glycerin fatty acid ester is used as a membrane for concentrating and separating bacterial cells.
Patent Document 6 discloses a microporous membrane made of a polyethylene resin having a viscosity average molecular weight of more than 1 million, containing at least one crystal component having a melting peak temperature of 145°C or higher, and having a porosity of 20 to 95% and an average pore diameter of 0.01 to 10 $\mu$m.
Patent Document 7 discloses a separation filter for medical use including a highly permeable microporous membrane made of a polyethylene resin and having a thickness of more than 25 $\mu$m and equal to or less than 1 mm, an average pore diameter of 0.01 to 10 $\mu$m, and a structural factor F of $1.5 \times 10^7$ seconds$^{-2} \cdot$ m$^{-1} \cdot$ Pa$^{-2}$ or more.
Patent Document 8 discloses a method for removing an aggregate present in a fluid containing a biological product by flowing the fluid through a membrane filter pretreated with a surfactant.
Patent Document 9 discloses a method of capturing intraoral microorganisms in a test liquid on a filtration membrane and recovering the intraoral microorganisms.
Patent Document 10 discloses a hydrophilic composite porous membrane including a porous structural matrix made of a polyolefin and an ethylene/vinyl alcohol-based copolymer coating layer with which the pore surface of the matrix is coated.
Patent Document 1: Japanese Patent Application Laid-Open (JP-A) No. H11-090184
Patent Document 2: Japanese National-Phase Publication (JP-A) No. 2013-531236
Patent Document 3: Japanese Patent Application Laid-Open (JP-A) No. 2009-183804
Patent Document 4: Japanese Patent Application Laid-Open (JP-A) No. 2003-268152
Patent Document 5: Japanese Patent Application Publication Laid-Open (JP-A) No. H06-057143
Patent Document 6: Japanese Patent Application Laid-Open (JP-A) No. 2004-016930
Patent Document 7: Japanese Patent Application Laid-Open (JP-A) No. 2002-265658
Patent Document 8: Japanese National-Phase Publication (JP-A) No. 2016-534748
Patent Document 9: Japanese Patent Application Laid-Open (JP-A) No. 2006-71478
Patent Document 10: Japanese Patent Application Laid-Open (JP-A) No. S61-271003

SUMMARY OF INVENTION

Technical Problem

For example, for the purpose of diagnosis of infection, viruses or bacteria may be separated from a specimen collected from an organism. One of methods for separating viruses or bacteria is a centrifugal separation method, and the centrifugal separation method is a method that requires equipment, labor, and time for repeating a centrifugal separation operation while changing a centrifugal force, centrifuging a sample in a buffer solution having a density gradient, or performing ultra-centrifugal separation. In addition, there is a method of separating viruses or bacteria using a porous membrane as one of the methods for separating viruses or bacteria. However, the conventional method is intended only to completely remove viruses and the like from a filtrate, or involves a complicated technique in which viruses and the like are adsorbed onto a membrane and taken out through a backwashing treatment, and thus there is no viewpoint of efficiently concentrating and recovering the viruses and the like in a specimen.

An embodiment of the present disclosure has been made under the above circumstances.

An object of an embodiment of the present disclosure is to provide a concentration membrane, a concentration device, a concentration system and a concentration method which are capable of easily and rapidly concentrating biological particles efficiently, and a method for detecting the biological particles.

Solution to Problem

Specific means for solving the problem include the following aspects.

[A1] A concentration membrane for use in concentrating biological particles, containing: a hydrophilic composite porous membrane including: a porous substrate; and a hydrophilic resin with which at least one main surface and inner surfaces of pores of the porous substrate are coated, the hydrophilic composite porous membrane having a ratio t/x of a membrane thickness t ($\mu$m) to an average pore diameter x ($\mu$m), as measured with a perm porometer, of from 50 to 630.

[A2] The concentration membrane according to [A1], wherein the average pore diameter x of the hydrophilic composite porous membrane is from 0.1 $\mu$m to 0.5 $\mu$m.

[A3] The concentration membrane according to [A1] or [A2], wherein the hydrophilic composite porous membrane has a bubble point pore diameter y of more than 0.8 $\mu$m and equal to or less than 3 $\mu$m, as measured with a perm porometer.

[A4] The concentration membrane according to any of [A1] to [A3], wherein the hydrophilic composite porous membrane has a ratio f/y of a water flow rate f (mL/(min $\cdot$ cm$^2$ $\cdot$ MPa)) to a bubble point pore diameter y ($\mu$m), as measured with a perm porometer, of from 100 to 480.

[A5] The concentration membrane according to any of [A1] to [A4], wherein the membrane thickness t of the hydrophilic composite porous membrane is from 10 $\mu$m to 150 $\mu$m.

[A6] The concentration membrane according to any of [A1] to [A5], wherein the hydrophilic composite porous membrane has a surface roughness Ra of from 0.3 $\mu$m to 0.7 $\mu$m.

[A7] The concentration membrane according to any of [A1] to [A6], wherein the hydrophilic resin comprises a hydrophilic resin in which a polymer main chain is composed only of a carbon atom and a side chain has at least one functional group selected from the group consisting of a hydroxy group, a carboxy group, and a sulfo group.

[A8] The concentration membrane according to any of [A1] to [A7], wherein the hydrophilic resin comprises at least one hydrophilic resin selected from the group consisting of polyvinyl alcohol, an olefin/vinyl alcohol-based resin, an acryl/vinyl alcohol-based resin, a methacryl/vinyl alcohol-based resin, a vinyl pyrrolidone/vinyl alcohol-based resin, polyacrylic acid, polymethacrylic acid, a perfluorosulfonic acid resin, and polystyrene sulfonic acid.

[A9] The concentration membrane according to any of [A1] to [A8], wherein the hydrophilic resin comprises an olefin/vinyl alcohol-based resin.

[A10] The concentration membrane according to any of [A1] to [A9], wherein the porous substrate is a polyolefin microporous membrane.

[A11] The concentration membrane according to any of [A1] to [A10], wherein the biological particles are 10 nm to 1,000 nm in size.

[A12] The concentration membrane according to any of [A1] to [A11], wherein the biological particles are viruses, bacteria, or exosomes.

[B1] A concentration device for biological particles containing:

a housing having an inlet and an outlet, wherein, due to a differential pressure between the inlet and the outlet, a liquid to be treated containing biological particles and water is injected from the inlet and discharged from the outlet;

a concentration membrane provided to separate the inlet and the outlet from each other in the housing, the concentration membrane being a hydrophilic porous membrane onto which the biological particles are not adsorbed, the concentration membrane allowing an effluent, which is a liquid having a concentration that is a concentration of the biological particles subtracted from a concentration of the liquid to be treated, to permeate from a surface on a side of the inlet to a surface on a side of the outlet; and

a concentration space portion that is a space on an upstream side of the concentration membrane in the housing and that stores a concentrated liquid which is a liquid having a concentration that is a concentration of the biological particles added to a concentration of the liquid to be treated by the concentration membrane.

[B2] The concentration device for biological particles according to [B1], wherein, in the housing, a volume of the concentration space portion is from 0.05 cm$^3$ to 5 cm$^3$.

[B3] The concentration device for biological particles according to [B1] or [B2], wherein, in the housing, a filtration area of the concentration membrane is from 1 cm$^2$ to 20 cm$^2$ ・

[B4] The concentration device for biological particles according to any of [B1] to [B3], wherein, in the housing, an inner wall portion facing the concentration space portion is formed with a guide groove continuous from the inlet.

[B5] The concentration device for biological particles according to any of [B1] to [B4], wherein, in the housing, an inner wall portion facing the concentration space portion has a shape in which a diameter gradually increases from the inlet toward the concentration membrane.

[B6] The concentration device for biological particles according to any of [B1] to [B5], wherein the concentration membrane includes a hydrophilic composite porous membrane containing a porous substrate, and a hydrophilic resin with which at least one main surface and inner surfaces of pores of the porous substrate are coated.

[B7] The concentration device for biological particles according to [B6], wherein the porous substrate is a polyolefin microporous membrane.

[B8] The concentration device for biological particles according to [B6] or [B7], wherein the hydrophilic resin comprises a hydrophilic resin in which a polymer main chain is composed only of a carbon atom and a side chain has at least one functional group selected from the group consisting of a hydroxy group, a carboxy group, and a sulfo group.

[B9] The concentration device for biological particles according to any of [B6] to [B8], wherein the hydrophilic resin comprises at least one hydrophilic resin selected from the group consisting of polyvinyl alcohol, an olefin/vinyl alcohol-based resin, an acryl/vinyl alcohol-based resin, a methacryl/vinyl alcohol-based resin, a vinyl pyrrolidone/vinyl alcohol-based resin, polyacrylic acid, polymethacrylic acid, a perfluorosulfonic acid resin, and polystyrene sulfonic acid.

[|310] The concentration device for biological particles according to any of [B6] to [B9], wherein the hydrophilic resin comprises an olefin/vinyl alcohol-based resin.

[B11] The concentration device for biological particles according to any of [B1] to [B10], wherein the concentration membrane has a ratio t/x of a membrane thickness t ($\mu$m) to an average pore diameter x ($\mu$m), as measured with a perm porometer, of from 50 to 630.

[B12] The concentration device for biological particles according to any of [B1] to [B11], wherein the membrane thickness t of the concentration membrane is from 10 $\mu$m to 150 $\mu$m.

[B13] The concentration device for biological particles according to any of [B1] to [B12], wherein the average pore diameter x, as measured with a perm porometer, of the concentration membrane is from 0.1 $\mu$m to 0.5 $\mu$m.

[B14] The concentration device for biological particles according to any of [B1] to [B13], wherein the concentration membrane has a bubble point pore diameter y of more than 0.8 $\mu$m and equal to or less than 3 $\mu$m, as measured with a perm porometer.

[B15] The concentration device for biological particles according to any of [B1] to [B14], wherein the concentration membrane has a ratio f/y of a water flow rate f (mL/(min・cm$^2$・MPa)) to a bubble point pore diameter y ($\mu$m), as measured with a perm porometer, of from 100 to 480.

[B16] The concentration device for biological particles according to any of [B1] to [B15], wherein the concentration membrane has a surface roughness Ra of from 0.3 $\mu$m to 0.7 $\mu$m.

[B17] A concentration system for biological particles including: the concentration device for biological particles according to any of [B1] to [B16]; and a unit for applying a differential pressure between the inlet and the outlet.

[B18] A method for concentrating biological particles, including steps of: supplying the liquid to be treated to the concentration device for biological particles according to any of [B1] to [B16]; applying a differential pressure between the inlet and the outlet of the concentration device to obtain the concentrated liquid in the concentration space portion; and recovering the concentrated liquid from the concentration space portion.

[B19] A method for detecting biological particles including steps of: supplying the liquid to be treated to the concentration device for biological particles according to any of [B1] to [B16]; applying a differential pressure between the inlet and the outlet of the concentration device to obtain the concentrated liquid in the concentration space portion; recovering the concentrated liquid from the concentration space portion; and detecting the biological particles contained in the collected concentrated liquid.

Advantageous Effects of Invention

According to an embodiment of the present disclosure, there are provided a concentration membrane, a concentration device, a concentration system, and a concentration method, which are capable of easily and rapidly concentrating biological particles efficiently, and a method for detecting the biological particles.

BRIEF DESCRIPTION OF DRAWINGS

Fig. 1 is a perspective view schematically showing an example of a concentration device for biological particles according to the present disclosure.

Fig. 2 schematically shows a cross section of the concentration device in Fig. 1.

Fig. 3 schematically shows a state where a liquid to be treated is supplied from an inlet in the concentration device in Fig. 2.

Fig. 4 schematically illustrates a state where a differential pressure is applied between the inlet and an outlet from the state in Fig. 3.

Fig. 5 schematically shows a state where a concentrated liquid is obtained from the state in Fig. 4.

Fig. 6 schematically shows a state where the concentrated liquid is recovered from the state in Fig. 5.

Fig. 7 is a perspective view schematically showing another example of an overall shape of a housing.

Fig. 8 is a perspective view schematically showing another example of the overall shape of the housing.

Fig. 9 is a perspective view schematically showing another example of the overall shape of the housing.

Fig. 10 is a perspective view schematically showing another example of the overall shape of the housing.

Fig. 11 is a perspective view schematically showing another example of a shape of the inlet.

Fig. 12 is a perspective view schematically showing another example of the shape of the inlet.

Fig. 13 is a perspective view schematically showing another example of the shape of the inlet.

Fig. 14 is a perspective view schematically showing another example of the shape of the inlet.

Fig. 15 is a perspective view schematically showing another example of a shape of the outlet.

Fig. 16 is a perspective view schematically showing another example of the shape of the outlet.

Fig. 17 is a perspective view schematically showing another example of the shape of the outlet.

Fig. 18 is a perspective view schematically showing another example of the shape of the outlet.

Fig. 19 is a perspective view schematically showing another example of a positional relationship between the inlet and the outlet.

Fig. 20 is a perspective view schematically showing another example of the positional relationship between the inlet and the outlet.

Fig. 21 is a perspective view schematically showing another example of the positional relationship between the inlet and the outlet.

Fig. 22 is a perspective view schematically showing another example of a shape of an internal space of the housing.

Fig. 23 schematically illustrates a cross section of Fig. 22.

Fig. 24 is a perspective view schematically showing another example of the shape of the internal space of the housing.

Fig. 25 schematically illustrates a cross section of Fig. 24.

Fig. 26 is a perspective view schematically showing another example of the shape of the internal space of the housing.

Fig. 27 is a perspective view schematically showing another example of the shape of the internal space of the housing.

Fig. 28 schematically illustrates a cross section of Fig. 27.

Fig. 29 is a perspective view schematically showing another example of the shape of the internal space of the housing.

Fig. 30 schematically illustrates a cross section of Fig. 29.

Fig. 31 is a perspective view schematically showing an example of a method for recovering a concentrated liquid.

Fig. 32 is a perspective view schematically showing another example of the method for recovering a concentrated liquid.

Fig. 33 is a perspective view schematically showing a state where the concentrated liquid is recovered from the state in Fig. 32.

Fig. 34 is a perspective view schematically showing an example of a shape of a concentration membrane 30.

Fig. 35 is a perspective view schematically showing another example of the shape of the concentration membrane 30.

Fig. 36 is a perspective view schematically showing another example of the shape of the concentration membrane 30.

Fig. 37 is a perspective view schematically showing another example of the shape of the concentration membrane 30.

Fig. 38 is a perspective view schematically showing an example of a concentration system.

Fig. 39 is a perspective view schematically showing another example of the concentration system.

Fig. 40 is a perspective view schematically showing another example of the concentration system.

Fig. 41 is a perspective view schematically showing another example of the concentration system.

Fig. 42 is a perspective view schematically showing another example of the concentration system.

Fig. 43 is a perspective view schematically showing another example of the concentration system.

Fig. 44 is a schematic diagram showing an instrument and an operation for a concentration test.

DESCRIPTION OF EMBODIMENTS

Hereinafter, embodiments of the invention will be described. These descriptions and examples illustrate embodiments and do not limit the scope of the invention. The mechanism of action described in the present disclosure includes presumptions, and whether or not the presumptions are correct does not limit the scope of the invention.

When an embodiment is described with reference to the drawings in the present disclosure, the configuration of the embodiment is not limited to the configuration illustrated in the drawings. In addition, the sizes of members in each drawing are conceptual, and the relative relationship between the sizes of the members is not limited thereto.

In the present disclosure, a numerical range indicated using "to" indicates a range including numerical values described before and after "to" as a lower limit value and an upper limit value, respectively.

In the numerical ranges described in stages in the present disclosure, the upper limit value or the lower limit value described in one numerical range may be replaced with the upper limit value or the lower limit value of any other numerical range described in stages. In addition, in the numerical ranges described in the present disclosure, the upper limit values or the lower limit values of the numerical ranges may be replaced with values shown in Examples.

In the present disclosure, the term "step" includes not only an independent step but also a step that cannot be clearly distinguished from other steps as long as the intended purpose of step is achieved.

In the present disclosure, each component may contain a plurality of corresponding substances. When referring to the amount of each component in a composition in the present disclosure, if there are a plurality of substances corresponding to each component in the composition, the amount means a total amount of the plurality of substances present in the composition unless otherwise specified.

In the present disclosure, "(meth)acryl" means at least one of acryl or methacryl, and "(meth)acrylate" means at least one of acrylate or methacrylate.

In the present disclosure, "monomer unit" means a constituent element of a polymer formed by polymerization of a monomer.

In the present disclosure, "machine direction" means an elongating direction in a membrane, film or sheet manufactured in an elongated shape, and "width direction" means a direction orthogonal to the "machine direction". In the present disclosure, the "machine direction" is also referred to as the "MD direction", and the "width direction" is also referred to as the "TD direction".

In the present disclosure, "main surface" of the membrane, film, or sheet means a wide outer surface other than the outer surface extending in a thickness direction, of the outer surfaces of the membrane, film, or sheet. The membrane, film or sheet includes two main surfaces. In the present disclosure, "side surface" of the membrane, film, or sheet refers to an outer surface extending in the thickness direction, of the outer surfaces of the membrane, film, or sheet.

In the present disclosure, with respect to the concentration membrane or the hydrophilic composite porous membrane, a side into which a liquid composition or a liquid to be treated flows is referred to as "upstream", and a side from which the liquid composition, the liquid to be treated or the effluent flows out is referred to as "downstream".

<Concentration membrane>

The present disclosure provides a concentration membrane for use in concentrating biological particles. The concentration membrane of the present disclosure is intended to treat a liquid composition containing water (hereinafter, referred to as an aqueous liquid composition), which may contain biological particles, and concentrates the liquid composition into an aqueous liquid composition having an increased concentration of biological particles.

The biological particles referred to in the present disclosure include particles possessed by an organism, particles released by an organism, particles parasitic on an organism, minute organisms, vesicles having a lipid as a membrane, and fragments thereof.

Examples of the biological particles referred to in the present disclosure include viruses, parts of viruses (e.g., particles obtained by removing an envelope from an enveloped virus), bacteriophages, bacteria, spores, cystoid spores, fungi, mold, yeast, cysts, protozoa, unicellular algae, plant cells, animal cells, cultured cells, hybridomas, tumor cells, blood cells, platelets, organelles (e.g., cell nuclei, mitochondria, vesicles), exosomes, apoptotic bodies, particles of lipid bilayers, particles of lipid monolayers, liposomes, aggregates of proteins, and fragments thereof. The biological particles referred to in the present disclosure also include artificial material.

The size of biological particles to be concentrated by the concentration membrane of the present disclosure is not limited. A diameter or long axis length of the biological particles is, for example, 1 nm or more, 5 nm or more, 10 nm or more, or 20 nm or more, and, for example, 100 $\mu$m or less, 50 $\mu$m or less, 1,000 nm or less, or 800 nm or less.

When a porous substrate of a hydrophilic composite porous membrane included in the concentration membrane of the present disclosure is a polyolefin microporous membrane (described later), it is appropriate that biological particles to be concentrated by the concentration membrane have a nano-order size. In this case, the diameter or long axis length of the biological particles is, for example, 10 nm or more, or 20 nm or more, and, for example, 1,000 nm or less, 800 nm or less, or 500 nm or less.

When a porous substrate of a hydrophilic composite porous membrane included in the concentration membrane of the present disclosure is a polyolefin microporous membrane, the concentration membrane is suitable for concentrating viruses, bacteria, or exosomes.

Examples of the aqueous liquid composition that serves as a sample by the concentration membrane of the present disclosure include animal (particularly, human) body fluids (for example, blood, serum, plasma, spinal fluid, tears, sweat, urine, pus, nasal mucus, sputum); dilutions of animal (particularly, human) body fluids; liquid compositions obtained by suspending excrement (for example, feces) of an animal (particularly, human) in water; gargling liquids for animals (particularly, human); buffer solutions containing extracts from an organ, a tissue, a mucous membrane, a skin, a squeezed specimen, a swab, and the like of animals (particularly, human); tissue extracts of marine products; water taken from aquaculture ponds for marine products; plant surface swabs or tissue extracts; soil extracts; extracts from plants; extracts from foods; and raw material liquids for pharmaceuticals.

The concentration membrane of the present disclosure includes a hydrophilic composite porous membrane containing a porous substrate, and a hydrophilic resin with which at least one main surface and inner surfaces of pores of the porous substrate are coated. The concentration membrane of the present disclosure may include a member other than the hydrophilic composite porous membrane. Examples of the member other than the hydrophilic composite porous membrane include a sheet-like reinforcing member disposed in contact with a part or all of a main surface or a side surface of the hydrophilic composite porous membrane; and a guide member for mounting the concentration membrane in a concentration device.

In the concentration membrane of the present disclosure, at least the main surface on the upstream side during the concentration treatment may be coated with the hydrophilic resin, and both the main surfaces are preferably coated with the hydrophilic resin.

In the hydrophilic composite porous membrane, exemplary embodiments in which the main surface of the porous substrate is coated with the hydrophilic resin include an embodiment in which the main surface of the porous substrate is partially or wholly coated with the hydrophilic resin, an embodiment in which openings of the porous substrate are partially or wholly filled with the hydrophilic resin, and an embodiment in which the main surface of the porous substrate is partially coated with the hydrophilic resin and the openings are partially filled with the hydrophilic resin. When the openings of the porous substrate are filled with the hydrophilic resin, the hydrophilic resin preferably forms a porous structure. Here, the porous structure means a structure in which a large number of micropores are provided inside, the micropores are coupled to each other, and a gas or a liquid can pass from one side to the other side.

In the hydrophilic composite porous membrane, exemplary embodiments in which the inner surfaces of pores of the porous substrate is coated with the hydrophilic resin include an embodiment in which wall surfaces of pores of the porous substrate are partially or wholly coated with the hydrophilic resin, an embodiment in which the pores of the porous substrate are partially or wholly filled with the hydrophilic resin, and an embodiment in which the wall surfaces of the pores of the porous substrate are partially coated with the hydrophilic resin and the pores are partially filled with the hydrophilic resin. When the pores of the porous substrate are filled with the hydrophilic resin, the hydrophilic resin preferably forms a porous structure. Here, the porous structure means a structure in which a large number of micropores are provided inside, the micropores are coupled to each other, and a gas or a liquid can pass from one side to the other side.

Concentration of the biological particles using the concentration membrane of the present disclosure is performed such that, when the aqueous liquid composition is allowed to pass one main surface to the other main surface of the hydrophilic composite porous membrane, some or all of the biological particles contained in the aqueous liquid composition do not pass through the hydrophilic composite porous membrane but remain in the aqueous liquid composition at at least any site of the upstream, the upstream-side main surface, and the inside of the pores of the hydrophilic composite porous membrane. A comparison is made between the aqueous liquid composition before the concentration treatment and the aqueous liquid composition recovered from at least any site of the upstream, the upstream-side main surface, and the inside of the pores of the hydrophilic composite porous membrane after the concentration treatment. When the concentration of the biological particles contained in the latter aqueous liquid composition is higher, the biological particles can be said to have been concentrated. A concentration rate (determined from the following formula) of the biological particles achieved by the concentration membrane of the present disclosure is more than 100%, preferably 200% or more, and more preferably 300% or more.

$$\text{Concentration rate (\%)} = \text{"biological particle concentration of aqueous liquid}$$

composition recovered from at least any site of upstream, main surface on upstream side, and

inside of pores of hydrophilic composite porous membrane after concentration treatment" ÷

"biological particle concentration of aqueous liquid composition before concentration

$$\text{treatment"} \times 100$$

Although the detailed mechanism is not necessarily clear, it is presumed that, when the hydrophilic composite porous membrane of the concentration membrane of the present disclosure has the hydrophilic resin on the upstream-side main surface and the inner surfaces of the pores, the biological particles remained at at least any site of the upstream-side main surface, and the inside of the pores of the hydrophilic composite porous membrane are easily recovered, and thus that the concentration rate of the biological particles is improved.

The hydrophilic composite porous membrane of the concentration membrane of the present disclosure includes a porous substrate and a hydrophilic resin with which at least one main surface and inner surfaces of pores of the porous substrate are coated, and that has a ratio $t/x$ of a membrane thickness $t$ ($\mu$m) to an average pore diameter $x$ ($\mu$m), as measured with a perm porometer, is from 50 to 630.

When $t/x$ of the hydrophilic composite porous membrane is less than 50, the membrane thickness $t$ is too small for the average pore diameter $x$, or the average pore diameter $x$ is too large for the membrane thickness $t$, and thus the biological particles easily pass through the hydrophilic composite porous membrane, so that a residual rate of the biological particles remaining at at least any site of the upstream, the upstream-side main surface, and the inside of the pores of the hydrophilic composite porous membrane (hereinafter, simply referred to as "residual rate of the biological particles") is poor, and, as a result, the concentration rate of the biological particles is poor. From this viewpoint, $t/x$ is 50 or more, preferably 80 or more, and more preferably 100 or more.

When $t/x$ of the hydrophilic composite porous membrane is more than 630, the membrane thickness $t$ is too large for the average pore diameter $x$, or the average pore diameter $x$ is too small for the membrane thickness $t$, and thus the aqueous liquid composition is less likely to pass through the hydrophilic composite porous membrane, and it takes time for the aqueous liquid composition to pass through the hydrophilic composite porous membrane (that is, it takes time to concentrate the aqueous liquid composition). From this viewpoint, $t/x$ is 630 or less, preferably 600 or less, more preferably 500 or less, further preferably 400 or less, and still more preferably 240 or less.

When the concentration membrane of the present disclosure is used, the biological particles can be concentrated easily and rapidly as compared with when the centrifugal separation method is used. When the concentration membrane of the present disclosure is used, the biological particles can be concentrated rapidly and efficiently as compared with when conventional porous membranes are used.

Hereinafter, the hydrophilic composite porous membrane, the porous substrate, and the hydrophilic resin of the concentration membrane of the present disclosure will be described in detail.

[Hydrophilic composite porous membrane]

In the hydrophilic composite porous membrane, a contact angle of water as measured by the following measurement conditions is preferably 90 degrees or less on one side or both sides. The contact angle of water is preferably smaller. More preferably, the hydrophilic composite porous membrane is so hydrophilic that the contact angle of water, when attempted to be measured on one side or both sides under the following measurement conditions, cannot be measured because a water droplet penetrates into the membrane.

Here, the contact angle of water is a value as measured by the following measurement method. The porous membrane is left in an environment at a temperature of 25°C and a relative humidity of 60% for 24 hours or more to adjust the humidity. Thereafter, a water droplet of 1 $\mu$L of ion-exchanged water is dropped on the surface of the porous membrane with a syringe under an environment at the same temperature and the same humidity, and a contact angle 30 seconds after dropping of the water droplet is measured by a $\theta/2$ method using a fully automatic contact angle meter (Kyowa Interface Science Co., Ltd., model number: Drop Master DM 500).

The thickness $t$ of the hydrophilic composite porous membrane is preferably 10 $\mu$m or more, more preferably 15 $\mu$m or more, further preferably 20 $\mu$m or more, and still further preferably 30 $\mu$m or more from the viewpoint of increasing the strength of the hydrophilic composite porous membrane and the viewpoint of increasing the residual rate of the biological particles. The thickness $t$ of the hydrophilic composite porous membrane is preferably 150 $\mu$m or less, more preferably 100 $\mu$m or less, further preferably 80 $\mu$m or less, and still further preferably 70 $\mu$m or less from the viewpoint of shortening a time necessary for the aqueous liquid composition to pass through the hydrophilic composite

9

porous membrane (hereinafter, referred to as treatment time for the aqueous liquid composition).

The thickness t of the hydrophilic composite porous membrane is determined by measuring values at 20 points with a contact type membrane thickness meter and averaging the measured values.

The average pore diameter x of the hydrophilic composite porous membrane as measured with a perm porometer is preferably 0.1 $\mu$m or more, more preferably 0.15 $\mu$m or more, and further preferably 0.2 $\mu$m or more, from the viewpoint of shortening the treatment time for the aqueous liquid composition and the viewpoint of easily recovering the biological particles remaining in the pores of the hydrophilic composite porous membrane. The average pore diameter x of the hydrophilic composite porous membrane as measured with a perm porometer is preferably 0.5 $\mu$m or less, more preferably 0.45 $\mu$m or less, and further preferably 0.4 $\mu$m or less from the viewpoint of increasing the residual rate of the biological particles.

The average pore diameter x of the hydrophilic composite porous membrane as measured with a perm porometer is determined by a half dry method specified in ASTM E1294-89 using a perm porometer (PMI, model: CFP-1200 AEXL) and using Galwick (surface tension: 15.9 dyn/cm) manufactured by PMI as an immersion liquid. When only one main surface of the hydrophilic composite porous membrane is coated with the hydrophilic resin, the main surface coated with the hydrophilic resin is placed toward a pressurizing part of the perm porometer, and the measurement is performed.

A bubble point pore diameter y of the hydrophilic composite porous membrane as measured with a perm porometer is preferably more than 0.8 $\mu$m, more preferably 0.9 $\mu$m or more, and further preferably 1.0 $\mu$m or more, from the viewpoint of shortening the treatment time for the aqueous liquid composition and the viewpoint of easily recovering the biological particles remaining in the pores of the hydrophilic composite porous membrane. The bubble point pore diameter y of the hydrophilic composite porous membrane as measured with a perm porometer is preferably 3 $\mu$m or less, more preferably 2.5 $\mu$m or less, and further preferably 2.2 $\mu$m or less from the viewpoint of increasing the residual rate of the biological particles.

The bubble point pore diameter y of the hydrophilic composite porous membrane as measured with a perm porometer is determined by a bubble point method (ASTM F316-86 and JIS K3832:1990) using a perm porometer (PMI, model: CFP-1200 AEXL). However, the value is determined by changing the immersion liquid at the time of the test to Galwick (surface tension: 15.9 dyn/cm) manufactured by PMI. When only one main surface of the hydrophilic composite porous membrane is coated with the hydrophilic resin, the main surface coated with the hydrophilic resin is placed toward a pressurizing part of the perm porometer, and the measurement is performed.

A bubble point pressure of the hydrophilic composite porous membrane is, for example, 0.01 MPa or more and 0.20 MPa or less, or 0.02 MPa to 0.15 MPa.

In the present disclosure, the bubble point pressure of the hydrophilic composite porous membrane is a value determined by immersing the hydrophilic composite porous membrane in ethanol, performing a bubble point test according to the bubble point test method of JIS K3832:1990, while changing the liquid temperature at the time of the test to 24 $\pm$ 2°C and the applied pressure is increased at a pressure increase rate of 2 kPa/sec. When only one main surface of the hydrophilic composite porous membrane is coated with the hydrophilic resin, the main surface coated with the hydrophilic resin is placed toward a pressurizing part of the measuring apparatus, and the measurement is performed.

A water flow rate f (mL/(min • cm$^2$ • MPa)) of the hydrophilic composite porous membrane is preferably 20 or more, more preferably 50 or more, further preferably 100 or more from the viewpoint of shortening the treatment time for the aqueous liquid composition. The water flow rate f (mL/(min • cm$^2$ • MPa)) of the hydrophilic composite porous membrane is preferably 1,000 or less, more preferably 800 or less, and further preferably 700 or less from the viewpoint of increasing the residual rate of the biological particles.

The water flow rate f of the hydrophilic composite porous membrane is determined by allowing 100 mL of water to permeate a sample set on a liquid permeation cell having a constant liquid permeation area (cm$^2$) at a constant differential pressure (20 kPa), measuring a time (sec) necessary for 100 mL of water to permeate the sample, and subjecting the measured value to unit conversion. When only one main surface of the hydrophilic composite porous membrane is coated with the hydrophilic resin, water is allowed to permeate from the main surface coated with the hydrophilic resin to the main surface not coated with the hydrophilic resin, and the measurement is performed.

In the hydrophilic composite porous membrane, the ratio f/y of the water flow rate f (mL/(min • cm$^2$ • MPa)) to the bubble point pore diameter y ($\mu$m) is preferably 100 or more, more preferably 150 or more, and further preferably 200 or more, from the viewpoint of shortening the treatment time for the aqueous liquid composition. In the hydrophilic composite porous membrane, the ratio f/y of the water flow rate f (mL/(min • cm$^2$ • MPa)) to the bubble point pore diameter y ($\mu$m) is preferably 480 or less, more preferably 400 or less, and further preferably 350 or less, from the viewpoint of increasing the residual rate of the biological particles.

From the viewpoint of increasing a recovery rate of the biological particles, the hydrophilic composite porous membrane has a surface roughness Ra of preferably 0.3 $\mu$m or more, and more preferably 0.4 $\mu$m or more, at least on the main surface on the upstream side during the concentration treatment. From the viewpoint of increasing the residual rate of the remaining biological particles, the hydrophilic composite porous membrane has a surface roughness Ra of preferably 0.7 $\mu$m or less, and more preferably 0.6 $\mu$m or less, at least on the main surface on the upstream side during the concentration treatment.

The surface roughness Ra of the hydrophilic composite porous membrane is determined by measuring surface roughnesses at three random places on the surface of a sample in a non-contact manner using a light wave interference type surface roughness meter (Zygo Corporation, NewView 5032), and using analysis software for roughness evaluation.

A Gurley value (seconds/100 mL · $\mu$m) per unit thickness of the hydrophilic composite porous membrane is, for example, 0.001 to 5, 0.01 to 3, or 0.05 to 1. The Gurley value of the hydrophilic composite porous membrane is a value as measured according to JIS P8117:2009.

A porosity of the hydrophilic composite porous membrane is, for example, 70% to 90%, 72% to 89%, or 74% to 87%. The porosity of the hydrophilic composite porous membrane is determined according to the following calculation method. That is, regarding constituent material 1, constituent material 2, constituent materials 3,..., and constituent material n of the hydrophilic composite porous membrane, when masses of the respective constituent materials are $W_1$, $W_2$, $W_3$,..., and $W_n$ (g/cm$^2$), true densities of the constituent materials are $d_1$, $d_2$, $d_3$,..., and $d_n$ (g/cm$^3$), and the membrane thickness is t (cm), the porosity $\varepsilon$ (%) is determined according to the following formula.

$$\varepsilon = \left( 1 - \frac{\sum_{i=1}^{n} \frac{Wi}{di}}{t} \right) \times 100$$

The hydrophilic composite porous membrane is preferably less likely to curl from the viewpoint of handleability. From the viewpoint of suppressing curling of the hydrophilic composite porous membrane, both the main surfaces of the hydrophilic composite porous membrane are preferably coated with the hydrophilic resin.

[Porous substrate]

In the present disclosure, the porous substrate means a substrate having pores or voids therein. Examples of the porous substrate include a microporous membrane; and a porous sheet made of a fibrous material, such as a nonwoven fabric or paper. As the porous substrate, a microporous membrane is preferable from the viewpoint of thinning and strength of the concentration membrane of the present disclosure. The microporous membrane means a membrane having a structure in which a large number of micropores are provided inside and the micropores are coupled to each other, and through which a gas or a liquid can pass from one surface to the other surface.

The material of the porous substrate may be either an organic material or an inorganic material.

The porous substrate may be either hydrophilic or hydrophobic. The concentration membrane of the present disclosure exhibits hydrophilicity because the porous substrate is coated with the hydrophilic resin even if the porous substrate is hydrophobic.

One embodiment of the porous substrate is a microporous membrane made of a resin. Examples of the resin constituting the microporous membrane include polyesters such as polyethylene terephthalate; polyolefins such as polyethylene and polypropylene; and heat-resistant resins such as wholly aromatic polyamide, polyamideimide, polyimide, polyethersulfone, polysulfone, polyetherketone, and polyetherimide.

One embodiment of the porous substrate is a porous sheet made of a fibrous material, and examples thereof include a nonwoven fabric and paper. Examples of the fibrous material constituting the porous sheet include fibrous materials of polyesters such as polyethylene terephthalate; fibrous material of polyolefins such as polyethylene and polypropylene; fibrous materials of heat-resistant resins such as wholly aromatic polyamide, polyamideimide, polyimide, polyethersulfone, polysulfone, polyetherketone, and polyetherimide; and fibrous materials of cellulose.

The surface of the porous substrate may be subjected to various surface treatments for the purpose of improving the wettability of a coating liquid used for coating the porous substrate with the hydrophilic resin. Examples of the surface treatment for the porous substrate include a corona treatment, a plasma treatment, a flame treatment, and an ultraviolet irradiation treatment.

[Physical properties of porous substrate]

The thickness of the porous substrate is preferably 10 $\mu$m or more, more preferably 15 $\mu$m or more, and further preferably 20 $\mu$m or more from the viewpoint of increasing the strength of the porous substrate and the viewpoint of increasing the residual rate of the biological particles. The thickness of the porous substrate is preferably 150 $\mu$m or less, more preferably 120 $\mu$m or less, further preferably 100 $\mu$m or less from the viewpoint of shortening the treatment time for the aqueous liquid composition. The method for measuring the thickness of the porous substrate is the same as the method for measuring the thickness t of the hydrophilic composite porous membrane.

The average pore diameter of the porous substrate as measured with a perm porometer is preferably 0.1 $\mu$m or more, more preferably 0.15 $\mu$m or more, and further preferably 0.2 $\mu$m or more, from the viewpoint of shortening the treatment time for the aqueous liquid composition and the viewpoint of easily recovering the biological particles remaining in the pores of the hydrophilic composite porous membrane. The average pore diameter of the porous substrate measured with the perm porometer is preferably 0.8 $\mu$m or less, more preferably 0.7 $\mu$m or less, and further preferably 0.6 $\mu$m or less from the viewpoint of increasing the residual rate of the biological particles. The average pore diameter of the porous substrate measured with the perm porometer is a value determined by a half dry method defined in ASTM E 1294-89 using a perm porometer, and the details of the measurement method are the same as the measurement method related to the average pore diameter x of the hydrophilic composite porous membrane.

The bubble point pore diameter of the porous substrate as measured with the perm porometer is preferably more than 0.8 $\mu$m, more preferably 0.9 $\mu$m or more, and further preferably 1.0 $\mu$m or more, from the viewpoint of shortening the treatment time for the aqueous liquid composition and the viewpoint of easily recovering the biological particles remaining in the pores of the hydrophilic composite porous membrane. The bubble point pore diameter of the porous substrate as measured with the perm porometer is preferably 3 $\mu$m or less, more preferably 2.8 $\mu$m or less, and further preferably 2.5 $\mu$m or less from the viewpoint of increasing the residual rate of the biological particles. The bubble point pore diameter of the porous substrate as measured with the perm porometer is a value determined by the bubble point method defined in ASTM F 316-86 and JIS K 3832 using a perm porometer, and the details of the measurement method are the same as those of the measurement method for the bubble point pore diameter y of the hydrophilic composite porous membrane.

The water flow rate (mL/(min • cm$^2$ • MPa)) of the porous substrate is preferably 20 or more, more preferably 50 or more, and further preferably 100 or more from the viewpoint of shortening the treatment time for the aqueous liquid composition. The water flow rate (mL/(min • cm$^2$ • MPa)) of the porous substrate is preferably 1,000 or less, more preferably 800 or less, and further preferably 700 or less from the viewpoint of increasing the residual rate of the biological particles. The method for measuring the water flow rate of the porous substrate is the same as the method for measuring the water flow rate f of the hydrophilic composite porous membrane. However, when the porous substrate is hydrophobic, the porous substrate immersed in ethanol in advance and dried at room temperature is used as a sample, and the sample set on the liquid permeation cell is wetted with a small amount (0.5 ml) of ethanol, then the measurement is performed.

The porous substrate has a surface roughness Ra of preferably 0.3 $\mu$m or more, and more preferably 0.4 $\mu$m or more on one side or both sides. The porous substrate has a surface roughness Ra of preferably 0.7 $\mu$m or less, and more preferably 0.6 $\mu$m or less on one side or both sides. The surface roughness Ra of the porous substrate is the same as the method for measuring the surface roughness Ra of the hydrophilic composite porous membrane.

A Gurley value (seconds/100 mL • $\mu$m) per unit thickness of the porous substrate is, for example, 0.001 to 5, preferably 0.01 to 3, and more preferably 0.05 to 1. The Gurley value of the porous substrate is a value as measured according to JIS P8117:2009.

A porosity of the porous substrate is, for example, 70% to 90%, preferably 72% to 89%, and more preferably 74% to 87%. The porosity of the porous substrate is determined according to the following calculation method. That is, regarding constituent material 1, constituent material 2, constituent materials 3,..., and constituent material n of the hydrophilic composite porous membrane, when masses of the respective constituent materials are $W_1$, $W_2$, $W_3$,..., and $W_n$ (g/cm$^2$), true densities of the constituent materials are $d_1$, $d_2$, $d_3$,..., and $d_n$ (g/cm$^3$), and the membrane thickness is t (cm), the porosity $\varepsilon$ (%) is determined according to the following formula.

$$\varepsilon = \left(1 - \frac{\sum_{i=1}^{n} \frac{Wi}{di}}{t}\right) \times 100$$

A BET specific surface area of the porous substrate is, for example, 1 m$^2$/g to 40 m$^2$/g, preferably 2 m$^2$/g to 30 m$^2$/g, and more preferably 3 m$^2$/g to 20 m$^2$/g. The BET specific surface area of the porous substrate is a value determined by measuring an adsorption isotherm at a set relative pressure of $1.0 \times 10^{-3}$ to 0.35 by a nitrogen gas adsorption method at a liquid nitrogen temperature using a specific surface area measuring apparatus (model: BELSORP-mini) manufactured by MicrotracBEL Corporation, and analyzing the adsorption isotherm by a BET method.

[Polyolefin microporous membrane]

One embodiment of the porous substrate is a microporous membrane containing polypropylene (referred to as a polyethylene microporous membrane in the present disclosure). The polyolefin contained in the polyolefin microporous membrane is not particularly limited, and examples thereof include polyethylene, polypropylene, polybutylene, polymethylpentene, and a copolymer of polypropylene and polyethylene. Among them, polyethylene is preferable, and high-density polyethylene, a mixture of high-density polyethylene and ultra-high molecular weight polyethylene, and the like are suitable. One embodiment of the polyolefin microporous membrane is a polyethylene microporous membrane containing only polyethylene as the polyolefin.

A weight average molecular weight (Mw) of the polyolefin contained in the polyolefin microporous membrane is, for example, 100,000 to 5 million. When the Mw of the polyolefin is 100,000 or more, sufficient mechanical characteristics can be imparted to the microporous membrane. When the Mw of the polyolefin is 5 million or less, the microporous membrane is easily molded.

One embodiment of the polyolefin microporous membrane is a microporous membrane containing a polyolefin composition (in the present disclosure, which means a mixture of polyolefins containing two or more polyolefins, and is referred to as a polyethylene composition when the polyolefin contained is only polyethylene). The polyolefin composition has an effect of forming a network structure with fibrillation during stretching and increasing the porosity of the polyolefin microporous membrane.

The polyolefin composition contains ultra-high molecular weight polyethylene having a weight average molecular weight of $9 \times 10^5$ or more in an amount of preferably 5% by mass to 40% by mass, more preferably 10% by mass to 35% by mass, and further preferably 15% by mass to 30% by mass, based on the total amount of the polyolefin.

The polyolefin composition is preferably a polyolefin composition obtained by mixing ultra-high molecular weight polyethylene having a weight average molecular weight of $9 \times 10^5$ or more and high-density polyethylene having a weight average molecular weight of $2 \times 10^5$ to $8 \times 10^5$ and a density of 920 kg/m$^3$ to 960 kg/m$^3$ at a mass ratio of 5:95 to 40:60 (more preferably 10:90 to 35:65, and even more preferably 15:85 to 30:70).

In the polyolefin composition, the weight average molecular weight of the entire polyolefin is preferably $2 \times 10^5$ to $2 \times 10^6$.

The weight average molecular weight of the polyolefin constituting the polyolefin microporous membrane is obtained by dissolving the polyolefin microporous membrane in o-dichlorobenzene under heating, and performing measurement by gel permeation chromatography (system: Alliance GPC 2000 manufactured by Waters Corporation, column: GMH6-HT and GMH6-HTL) under the conditions of a column temperature of 135°C and a flow rate of 1.0 mL/min. Molecular weight monodisperse polystyrene (manufactured by Tosoh Corporation) is used for calibration of the molecular weight.

One embodiment of the polyolefin microporous membrane is a microporous membrane containing polypropylene from the viewpoint of having heat resistance such that the polyolefin microporous membrane does not break easily when exposed to a high temperature.

One embodiment of the polyolefin microporous membrane is a polyolefin microporous membrane containing at least a mixture of polyethylene and polypropylene.

One embodiment of the polyolefin microporous membrane is a polyolefin microporous membrane having a laminated structure of two or more layers, in which at least one layer contains polyethylene and at least one layer contains polypropylene.

[Method for producing polyolefin microporous membrane]

The polyolefin microporous membrane can be produced, for example, by a production method including the following steps (I) to (IV):

Step (I): a step of preparing a solution containing a polyolefin composition and a volatile solvent having a boiling point of less than 210°C at atmospheric pressure;
Step (II): a step of melt-kneading the solution, extruding the obtained melt-kneaded product from a die, and cooling and solidifying the extrudate to obtain a first gel-like molded product;
Step (III): a step of stretching (primary stretching) the first gel-like molded product in at least one direction and drying the solvent to obtain a second gel-like molded product; and
Step (IV): a step of stretching (secondary stretching) the second gel-like molded product in at least one direction.

Step (I) is a step of preparing a solution containing a polyolefin composition and a volatile solvent having a boiling point of less than 210°C at atmospheric pressure. The solution is preferably a thermoreversible sol-gel solution, and the polyolefin composition is dissolved in a solvent under heating to be solated, thereby preparing a thermoreversible sol-gel solution. The volatile solvent having a boiling point of less than 210°C at atmospheric pressure is not particularly limited as long as it is a solvent capable of sufficiently dissolving the polyolefin. Examples of the volatile solvent include tetralin (206°C to 208°C), ethylene glycol (197.3°C), decalin (decahydronaphthalene, 187°C to 196°C), toluene (110.6°C), xylene (138°C to 144°C), diethyltriamine (107°C), ethylenediamine (116°C), dimethylsulfoxide (189°C), and hexane (69°C), and decalin or xylene is preferable (the temperatures in parentheses are their boiling points at atmospheric pressure). The volatile solvents may be used singly or, two or more thereof may be used in combination.

The polyolefin composition used in step (I) (in the present disclosure, which means a mixture of polyolefins containing two or more polyolefins, and is referred to as a polyethylene composition when the polyolefin contained is only polyethylene) preferably contains polyethylene, and more preferably is a polyethylene composition.

In the solution prepared in step (I), the concentration of the polyolefin composition is preferably 10% by mass to 40% by mass, and more preferably 15% by mass to 35% by mass from the viewpoint of controlling the porous structure of the polyolefin microporous membrane. When the concentration of the polyolefin composition is 10% by mass or more, the occurrence of cutting can be suppressed in the process for forming the polyolefin microporous membrane, and the dynamic strength of the polyolefin microporous membrane is increased to improve the handleability. When the concentration of the polyolefin composition is 40% by mass or less, pores of the polyolefin microporous membrane are easily formed.

Step (II) is a step of melt-kneading the solution prepared in step (I), extruding the obtained melt-kneaded product from a die, and cooling and solidifying the extrudate to obtain a first gel-like molded product. In Step (II), for example, the melt-kneaded product is extruded from a die in a temperature range from the melting point of the polyolefin composition to the melting point + 65°C to obtain an extrudate, and then the extrudate is cooled to obtain a first gel-like molded product. The first gel-like molded product is preferably shaped into a sheet. The cooling may be performed by immersion in water or an organic solvent, or may be performed by contact with a cooled metal roll, and is generally performed by immersion in the volatile solvent used in step (I).

Step (III) is a step of stretching (primary stretching) the first gel-like molded product in at least one direction and drying the solvent to obtain a second gel-like molded product. The stretching step in step (III) is preferably biaxial stretching, and may be sequential biaxial stretching in which longitudinal stretching and transverse stretching are separately performed, or simultaneous biaxial stretching in which longitudinal stretching and transverse stretching are simultaneously performed. A stretch ratio for the primary stretching (product of a longitudinal stretch ratio and a lateral stretch ratio) is preferably 1.1 times to 3 times, and more preferably 1.1 times to 2 times, from the viewpoint of controlling the porous structure of the polyolefin microporous membrane. The temperature during the primary stretching is preferably 75°C or lower. The drying step in step (III) is performed without any particular limitation as long as the drying temperature is a temperature at which the second gel-like molded product is not deformed, but is preferably performed at 60°C or lower.

The stretching step and the drying step in step (III) may be performed simultaneously or stepwise. For example, the primary stretching may be performed while preliminary drying may be performed, and then main drying may be performed. Alternatively, the primary stretching may be performed between the preliminary drying and the main drying. The primary stretching can also be performed in a state where drying is controlled and the solvent remains in a suitable state.

Step (IV) is a step of stretching (secondary stretching) the second gel-like molded product in at least one direction. The stretching step of step (IV) is preferably biaxial stretching. The stretching step of step (IV) may be any of: sequential biaxial stretching in which longitudinal stretching and transverse stretching are separately performed; simultaneous biaxial stretching in which longitudinal stretching and transverse stretching are simultaneously performed; a step of stretching the second gel-like molded product a plurality of times in the longitudinal direction and then stretching it in the lateral direction; a step of stretching the second gel-like molded product in the longitudinal direction and stretching it a plurality of times in the transverse direction; and a step of sequentially performing biaxial stretching and then further performing stretching once or a plurality of times in the longitudinal direction and/or the lateral direction.

A stretch ratio for the secondary stretching (product of the longitudinal stretch ratio and the lateral stretch ratio) is preferably 5 to 90 times, and more preferably 10 to 60 times, from the viewpoint of controlling the porous structure of the polyolefin microporous membrane. A stretching temperature for the secondary stretching is preferably 90°C to 135°C, and more preferably 90°C to 130°C from the viewpoint of controlling the porous structure of the polyolefin microporous membrane.

After step (IV), heat fixation treatment may be performed. A heat fixation temperature is preferably 110°C to 160°C, and more preferably 120°C to 150°C, from the viewpoint of controlling the porous structure of the polyolefin microporous membrane.

After the heat fixation treatment, the solvent remaining in the polyolefin microporous membrane may be further subjected to an extraction treatment and an annealing treatment. The extraction treatment for the remaining solvent is performed, for example, by immersing the sheet after the heat fixation treatment in a methylene chloride bath to elute the remaining solvent in methylene chloride. In the polyolefin microporous membrane immersed in the methylene chloride bath, methylene chloride is preferably removed by drying after the polyolefin microporous membrane is lifted from the methylene chloride bath. The annealing treatment is performed by conveying the polyolefin microporous membrane on a roller heated to, for example, 100°C to 140°C after the extraction treatment for the remaining solvent.

Each of the conditions in steps (I) to (IV) is controlled, thereby making it possible to produce a polyolefin microporous membrane having a ratio t/x of the membrane thickness t ($\mu$m) to the average pore diameter x ($\mu$m) of 50 to 630. For example, the ratio t/x can be controlled to 50 or more by decreasing the longitudinal stretch ratio. For example, the ratio t/x can be controlled to 630 or less by increasing the longitudinal stretch ratio.

[Hydrophilic resin]

The hydrophilic resin is not particularly limited, and examples thereof include resins having a hydrophilic group such as a hydroxy group, a carboxy group, or a sulfo group.

The hydrophilic resin is preferably a resin in which a polymer has a main chain composed only of carbon atoms and a side chain having at least one functional group selected from the group consisting of a hydroxy group, a carboxy group, and a sulfo group, from the viewpoint of difficulty in falling off from the porous substrate and concentration rate of the biological particles.

Examples of the hydrophilic resin include resins in which a polymer has a main chain containing not only a carbon atom but also an oxygen atom (for example, polyethylene glycol, cellulose, and the like), but the hydrophilic resin in which a polymer has a main chain containing an oxygen atom is relatively likely to fall off from the porous substrate. From the viewpoint of difficulty in falling off from the porous substrate, a resin in which a polymer has a main chain composed only of carbon atoms is preferable, and a resin in which a polymer has a main chain composed only of carbon atoms and that has a side chain having at least one functional group selected from the group consisting of a hydroxy group, a carboxy group, and a sulfo group is more preferable.

The hydrophilic resin preferably contains at least one hydrophilic resin selected from the group consisting of polyvinyl alcohol, an olefin/vinyl alcohol-based resin, an acryl/vinyl alcohol-based resin, a methacryl/vinyl alcohol-based resin, a vinyl pyrrolidone/vinyl alcohol-based resin, polyacrylic acid, polymethacrylic acid, a perfluorosulfonic acid resin, and polystyrene sulfonic acid. The hydrophilic resin more preferably contains an olefin/vinyl alcohol-based resin, among them.

Examples of the hydrophilic resin include a hydrophilic resin obtained by graft polymerization of a hydrophilic monomer on the surface of the porous substrate. In this case, the hydrophilic resin is directly chemically bonded to the surface of the porous substrate. Examples of the hydrophilic monomer graft-polymerized on the surface of the porous substrate include acrylic acid, methacrylic acid, vinyl alcohol, N-vinyl-2 pyrrolidone, and vinyl sulfonic acid. From the viewpoint of manufacturability of the hydrophilic composite porous membrane, a form in which the hydrophilic resin is attached to the surface of the porous substrate by a coating method or the like (a form in which the hydrophilic resin is not chemically bonded to the surface of the porous substrate) is more preferable than a form in which the hydrophilic resin is directly chemically bonded to the surface of the porous substrate as in graft polymerization.

The hydrophilic resin may be one kind or two or more kinds.

The hydrophilic resin is preferably the olefin/vinyl alcohol-based resin from the viewpoint of less irritation to the biological particles and the viewpoint of easily recovering the biological particles remaining on the upstream-side main surface and in the pores of the hydrophilic composite porous membrane.

Examples of the olefin constituting the olefin/vinyl alcohol-based resin include ethylene, propylene, butene, pentene, hexene, heptene, octene, nonene, and decene. The olefin is preferably an olefin having 2 to 6 carbon atoms, more preferably an $\alpha$-olefin having 2 to 6 carbon atoms, further preferably an $\alpha$-olefin having 2 to 4 carbon atoms, and particularly preferably ethylene. The olefin unit contained in the olefin/vinyl alcohol-based resin may be one kind or two or more kinds.

The olefin/vinyl alcohol-based resin may contain a monomer other than olefin and vinyl alcohol as a constituent unit. Examples of the monomer other than olefin and vinyl alcohol include at least one acrylic monomer selected from the group consisting of (meth)acrylic acid, (meth)acrylic acid salts, and (meth)acrylic acid esters; and styrene monomers such as styrene, meta-chlorostyrene, para-chlorostyrene, para-fluorostyrene, paramethoxystyrene, meta-tert-butoxystyrene, para-tert-butoxystyrene, para-vinylbenzoic acid, and para-methyl-$\alpha$-methylstyrene. One, or two or more of these monomer units may be contained in the olefin/vinyl alcohol-based resin.

The olefin/vinyl alcohol-based resin may contain a monomer other than olefin and vinyl alcohol as a constituent unit, but, from the viewpoint of less irritation to the biological particles and the viewpoint of easily recovering the biological particles remaining in the pores of the hydrophilic composite porous membrane, a total proportion of the olefin unit and the vinyl alcohol unit is preferably 85% by mol or more, more preferably 90% by mol or more, further preferably 95% by mol or more, and particularly preferably 100% by mol. As the olefin/vinyl alcohol-based resin, a binary copolymer of olefin and vinyl alcohol is preferable (here, preferred embodiments of the olefin are as described above), and a binary copolymer of ethylene and vinyl alcohol is more preferable.

A proportion of the olefin unit in the olefin/vinyl alcohol-based resin is preferably 20% by mol to 55% by mol.

When the proportion of the olefin unit is 20% by mol or more, the olefin/vinyl alcohol-based resin is less likely to be dissolved in water. From this viewpoint, the proportion of the olefin unit is more preferably 23% by mol or more, and further preferably 25% by mol or more. On the other hand, when the proportion of the olefin unit is 55% by mol or less, the olefin/vinyl alcohol-based resin has higher hydrophilicity. From this viewpoint, the proportion of the olefin unit is more preferably 52% by mol or less, and further preferably 50% by mol or less.

Examples of commercially available products of the olefin/vinyl alcohol-based resin include "Soarnol" series manufactured by The Nippon Synthetic Chemical Industry Co., Ltd. and "Eval" series manufactured by Kuraray Co., Ltd.

An amount of the hydrophilic resin adhered to the porous substrate is, for example, 0.01 $g/m^2$ to 5 $g/m^2$, 0.02 $g/m^2$ to 2 $g/m^2$, or 0.03 $g/m^2$ to 1 $g/m^2$. The amount of the hydrophilic resin adhered to the porous substrate is a value (Wa-Wb) obtained by subtracting a basis weight Wb ($g/m^2$) of the porous substrate from a basis weight Wa ($g/m^2$) of the hydrophilic composite porous membrane.

[Method for producing hydrophilic composite porous membrane]

The method for producing the hydrophilic composite porous membrane is not particularly limited. Examples of general production methods includes a method of applying a coating liquid containing an hydrophilic resin to a porous substrate, drying the coating liquid, and coating the porous substrate with the hydrophilic resin; and a method of graftpolymerizing a hydrophilic monomer on a porous substrate and coating the porous substrate with an hydrophilic resin.

The coating liquid containing an hydrophilic resin can be prepared by mixing and stirring the hydrophilic resin in a solvent having a temperature increased to a temperature equal to or higher than the melting point of the hydrophilic resin, thereby dissolving or dispersing the hydrophilic resin in the solvent. The solvent is not particularly limited as long as it is a good solvent for the hydrophilic resin, and specific examples thereof include a 1-propanol aqueous solution, a 2-propanol aqueous solution, an N,N-dimethylformamide aqueous solution, a dimethyl sulfoxide aqueous solution, and an ethanol aqueous solution. A ratio of the organic solvent in the aqueous solution is preferably in a range of 30% by mass to 70% by mass.

The concentration of the hydrophilic resin in the coating liquid when the coating liquid containing the hydrophilic resin is applied to the porous substrate is preferably 0.01% by mass to 5% by mass. When the concentration of the hydrophilic resin in the coating liquid is 0.01% by mass or more, hydrophilicity can be efficiently imparted to the porous substrate. From such a viewpoint, the concentration of the hydrophilic resin in the coating liquid is more preferably 0.05% by mass or more, and further preferably 0.1% by mass or more. When the concentration of the hydrophilic resin in the coating liquid is 5% by mass or less, the water flow rate in the produced hydrophilic composite porous membrane is large. From such a viewpoint, the concentration of the hydrophilic resin in the coating liquid is more preferably 3% by mass or less, and further preferably 2% by mass or less.

The application of the coating liquid to the porous substrate can be performed by known coating methods. Examples of the coating method include, for example, an immersion method, a knife coater method, a gravure coater method, a screen printing method, a Meyer bar method, a die coater method, a reverse roll coater method, an inkjet method, a spray method, and a roll coater method. In addition, by adjusting the temperature of the coating liquid at the time of coating, a layer of the hydrophilic resin can be stably obtained. Here, the temperature of the coating liquid is not particularly limited, but is preferably in a range of 5°C to 40°C.

The temperature at which the coating liquid is dried is preferably 25°C to 100°C. When the drying temperature is 25°C or higher, the time necessary for drying can be shortened. From such a viewpoint, the dry concentration is more preferably 40°C or higher, and further preferably 50°C or higher. On the other hand, when the drying temperature is 100°C or lower, shrinkage of the porous substrate is less likely to occur. From such a viewpoint, the drying temperature is more preferably 90°C or lower, and further preferably 80°C or lower.

The hydrophilic composite porous membrane may contain a surfactant, a wetting agent, an antifoaming agent, a pH adjusting agent, a coloring agent, and the like.

<Concentration device>

The present disclosure provides a concentration device for use in concentrating biological particles. The concentration device of the present disclosure is intended to treat a "liquid to be treated" which is a liquid containing water, which may contain biological particles, and concentrates the liquid to a "concentrated liquid" having an increased concentration of the biological particles.

Here, with respect to the liquid to be treated, "containing water" means that water is used as a solvent or a component, and the content thereof is not particularly limited. In addition, with respect to the liquid to be treated, "containing biological particles" refers to a state where the biological particles are floated, suspended, or precipitated without being dissolved in the liquid to be treated.

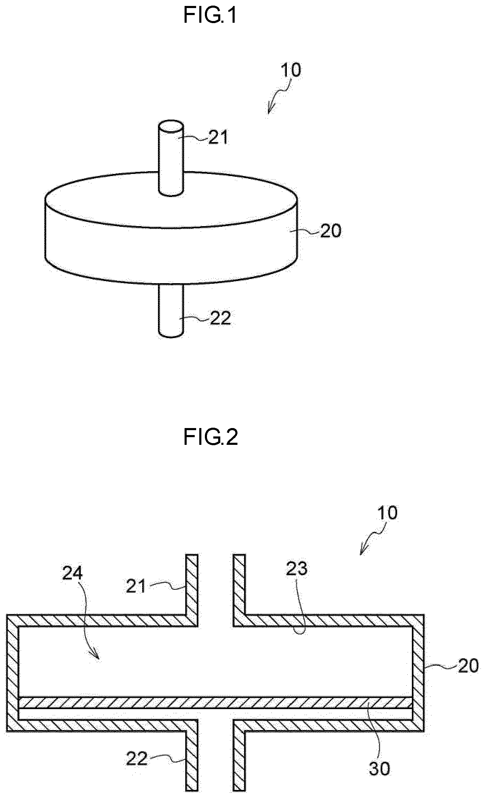

A concentration device 10 for biological particles 50 according to the present disclosure exhibits an appearance with an inlet 21 and an outlet 22 that open into a housing 20 having an internal space, as shown in the schematic perspective view of Fig. 1. In the figure, an example of a columnar shape having a diameter longer than its height is illustrated as a shape of the housing 20. More specifically, as illustrated in the schematic cross-sectional view of Fig. 2, in the internal space of the housing 20, the cylindrical inlet 21 protruding upward to the upstream side is opened, and the cylindrical outlet 22 protruding downward to the downstream side is opened. In the internal space of the housing 20, the inlet 21 and the outlet 22 are separated from each other by the concentration membrane 30. A space on an upstream side of the concentration membrane 30 in the housing 20 is a concentration space portion 24.

In other words, the housing 20 has the inlet 21 and the outlet 22. Further, due to a differential pressure between the inlet 21 and the outlet 22, a liquid to be treated 40 containing the biological particles 50 and water is injected from the inlet 21 and discharged from the outlet 22.

Further, the concentration membrane 30 is provided to separate the inlet 21 and the outlet 22 from each other in the housing 20. The concentration membrane 30 is a hydrophilic porous membrane onto which the biological particles 50 are not adsorbed, and allows an effluent 42, which is a liquid having a concentration that is a concentration of the biological particles subtracted from a concentration of the liquid to be treated 40, to permeate from a surface on a side of the inlet 21 to a surface on a side of the outlet 22.

The concentration space portion 24 is the space on the upstream side of the concentration membrane 30 in the housing 20 (in other words, a region defined by the inner wall portion 23 of the housing 20 and the upstream-side main surface of the concentration membrane 30). The concentration space portion 24 stores a concentrated liquid 41 which is a liquid having a concentration that is a concentration of the biological particles added to a concentration of the liquid to be treated 40 by the concentration membrane 30.

Examples of the liquid to be treated 40, which is injected into the concentration device 10 of the present disclosure include animal (particularly, human) body fluids (for example, blood, serum, plasma, spinal fluid, tears, sweat, urine, pus, nasal mucus, sputum); dilutions of animal (particularly, human) body fluids; liquid compositions obtained by suspending excrement (for example, feces) of an animal (particularly, human) in water; gargling liquids for animals (particularly, human); buffer solutions containing extracts from an organ, a tissue, a mucous membrane, a skin, a squeezed specimen, a swab, and the like of animals (particularly, human); tissue extracts of marine products; water taken from aquaculture ponds for marine products; plant surface swabs or tissue extracts; soil extracts; extracts from plants; extracts from foods; and raw material liquids for pharmaceuticals.

[Methods for concentrating and detecting biological particles 50]

A method for concentrating the biological particles 50 by the concentration device 10 of the present disclosure is as follows.

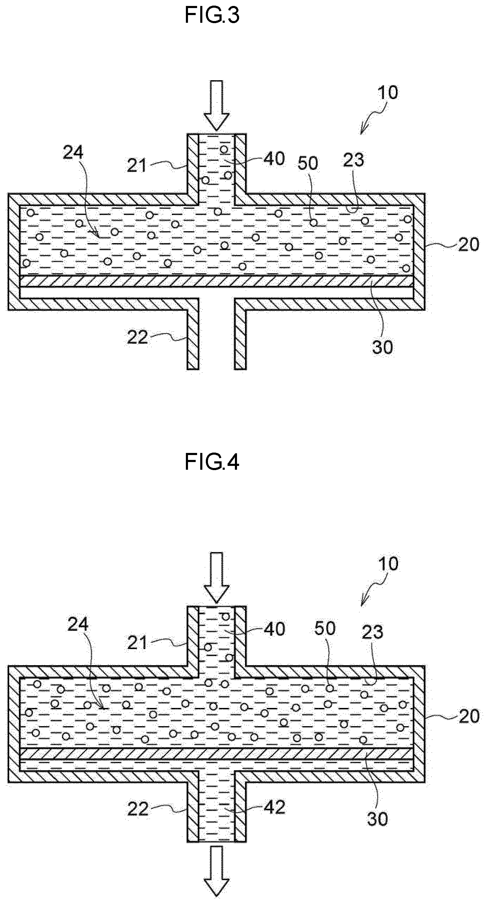

As illustrated in Fig. 3, a step of supplying the liquid to be treated 40 containing the biological particles 50 and water to the concentration device 10 from the inlet 21 is performed.

Next, a step of applying a differential pressure between the inlet 21 and the outlet 22 to obtain a concentrated liquid in the concentration space portion 24 is performed.

That is, by applying the differential pressure between the inlet 21 and the outlet 22 to the injected liquid to be treated 40, the effluent 42 that has permeated the concentration membrane 30 is discharged, as illustrated in Fig. 4. The differential pressure at this time can be generated by pressurization from the inlet 21, or depressurization from the outlet 22, or both. The effluent 42 has a concentration that is a concentration of the biological particles 50 subtracted from a concentration of the liquid to be treated 40, as described above.

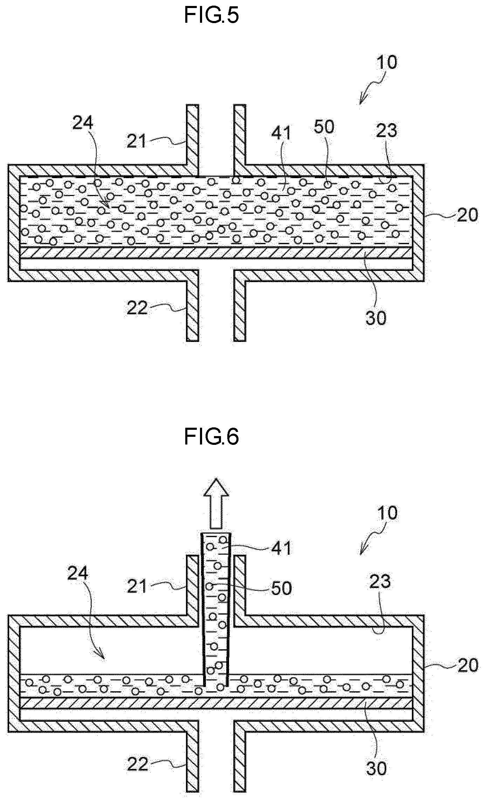

Then, as illustrated in Fig. 5, the concentrated liquid 41 is obtained in the concentration space portion 24. The concentrated liquid 41 has a concentration that is a concentration of the biological particles 50 added to a concentration of the liquid to be treated 40, as described above.

Next, a step of recovering the concentrated liquid 41 from the concentration space portion 24 is performed. That is, as illustrated in Fig. 6, the concentrated liquid 41 is recovered from the concentration space portion 24 using an appropriate tool or device such as a micropipette.

Finally, a step of detecting the biological particles 50 contained in the recovered concentrated liquid 41 is performed. From the recovered concentrated liquid 41, the biological particles 50 contained therein are detected by an appropriate means according to the kind and properties thereof. For example, in a case where a detection target for the biological particles 50 is a nucleic acid (DNA or RNA), a polymerase chain reaction (PCR), Southern blotting, Northern blotting, or the like is performed. In a case where the detection target for the biological particles 50 is a protein, mass spectrometry, Western blotting, immunochromatography, or the like is performed. In a case where the detection target for the biological particles 50 is a sugar or a lipid, mass spectrometry or the like is performed.

In the housing 20, a volume of the concentration space portion 24 can be appropriately determined according to the properties and amount of the liquid to be treated 40, but is desirably 0.05 $cm^3$ to 5 $cm^3$ in consideration of convenience of use.

In the housing 20, ae filtration area, which is a portion where the concentration membrane 30 is actually in contact with the liquid to be treated 40, can be appropriately determined according to the properties and amount of the liquid to be treated 40, but is desirably 1 cm$^2$ to 20 cm$^2$ in consideration of convenience of use.

<Overall shape of housing 20>

An overall shape of the housing 20 is not limited to the columnar shape as illustrated in Fig. 1, and may take various shapes.

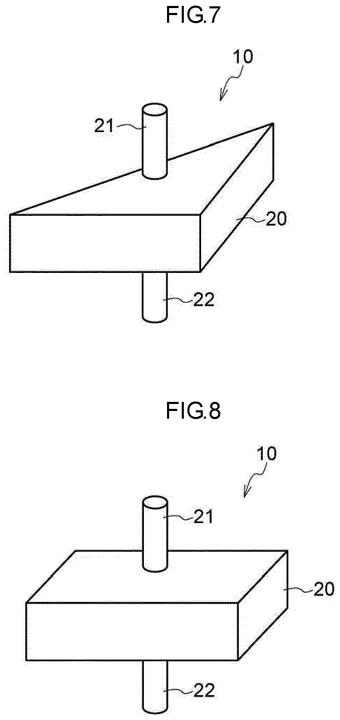

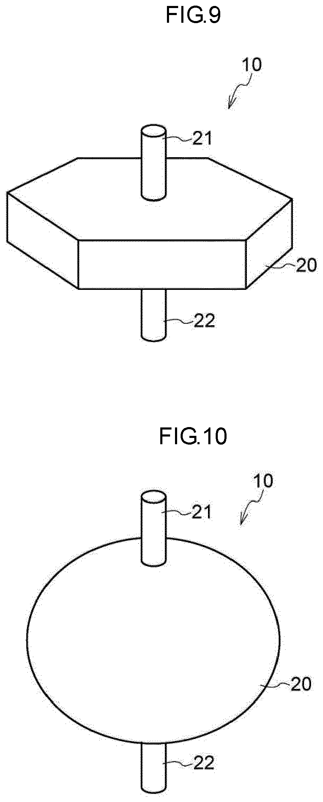

For example, the overall shape can be a triangular prism shape (Fig. 7), a quadrangular prism shape (Fig. 8), and any other polygonal prism shape, for example, a hexagonal prism shape (Fig. 9). In either case, the inlet 21 is provided on one (for example, an upper bottom surface) of both bottom surfaces of the prism shape, and the outlet 22 is provided on the other (for example, a lower bottom surface) thereof.

The overall shape of the housing 20 may be a spherical shape as illustrated in Fig. 10. Also in this case, the inlet 21 is provided at one pole (for example, a pole at an upper end) of the spherical shape, and the outlet 22 is provided at the other pole (for example, a pole at a lower end) thereof.

A material of the housing 20 is not particularly limited, but is desirably a synthetic resin, particularly, a polypropylene resin, a polyethylene resin, a vinyl chloride resin, a fluororesin, an ABS resin, an MBS resin, a polycarbonate resin, an acrylic resin, or a polystyrene resin. A method for molding the housing 20 is also not particularly limited, and the housing 20 can be formed by forming an upstream-side member including the inlet 21 and a downstream-side member including the outlet 22 by injection molding, and binding both the members by an appropriate method such as adhesion, welding, or screwing in a state where the concentration membrane 30 is sandwiched between these members.

<Shape of inlet 21>

A shape of the inlet 21 is not limited to the cylindrical shape protruding upward as illustrated in Fig. 1, and may take various shapes.

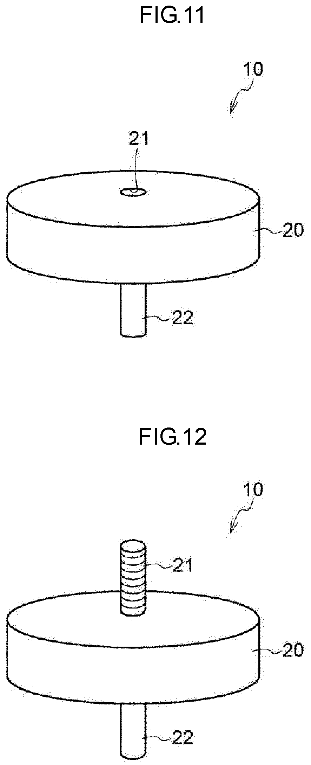

For example, as illustrated in Fig. 11, the inlet 21 can be formed as a simple hole without adopting the structure protruding upward. In this case, a transportation pipe for the liquid to be treated 40 can be inserted into the inlet 21.

As illustrated in Fig. 12, a screw groove (male screw) can be provided on an outer peripheral surface of the cylindrical inlet 21 protruding upward. In this case, it is possible to prevent detachment between a transportation path for the liquid to be treated 40 and the inlet 21, by providing a female screw which is screwed into the screw groove at a terminal end of the transportation path.

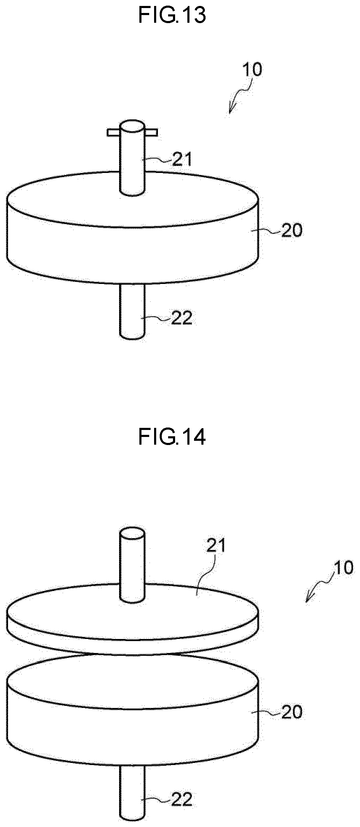

Furthermore, as illustrated in Fig. 13, a male side of a luer lock can be provided on a tip outer peripheral surface of the cylindrical inlet 21 protruding upward. In this case, it is possible to prevent the transportation path for the liquid to be treated 40 and the inlet 21 from coming off, by providing a female side which is fitted to the male side of the luer lock at a terminal end of the transportation path.

As illustrated in Fig. 14, the inlet 21 may also be formed in the shape of a lid that closes the housing 20 whose upper surface is opened, from above.

<Shape of outlet 22>

A shape of the outlet 22 is not limited to the cylindrical shape protruding downward as illustrated in Fig. 1, and may take various shapes.

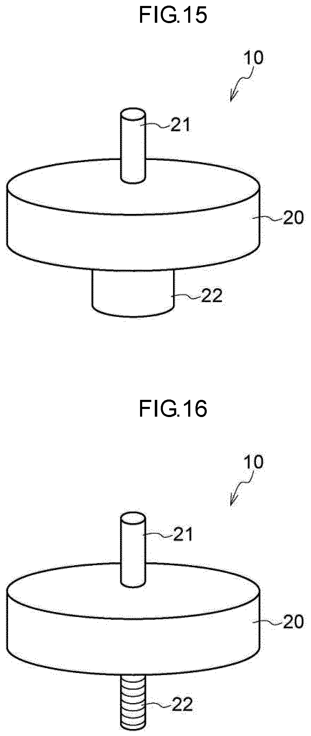

For example, as illustrated in Fig. 15, the outlet 22 may have a pipe diameter different from that of the inlet 21.

As illustrated in Fig. 16, a thread groove (male thread) may be provided on an outer peripheral surface of the cylindrical outlet 22 protruding downward. In this case, it is possible to prevent detachment between a recovery path for the effluent 42 and the outlet 22, by providing a female screw which is screwed into the screw groove at a tip of the recovery path.

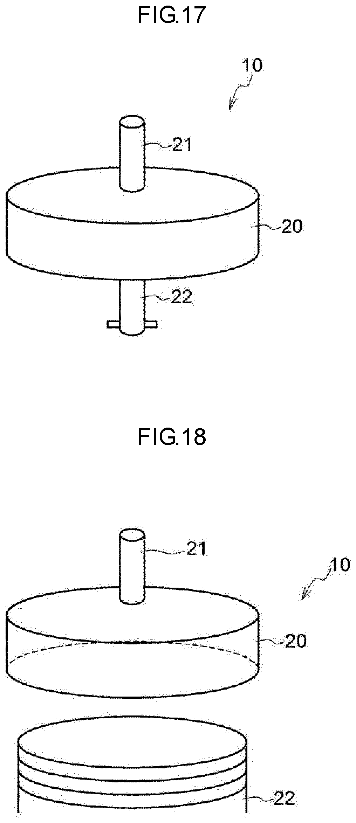

Furthermore, as illustrated in Fig. 17, a male side of a luer lock can be provided on a tip outer peripheral surface of the cylindrical outlet 22 protruding downward. In this case, it is possible to prevent detachment between the recovery path for the effluent 42 and the outlet 22, by providing a female side which is fitted to the male side of the luer lock at the tip of the recovery path.

As illustrated in Fig. 18, the outlet 22 may also be formed in the shape of a column that closes the housing 20 whose lower surface is open, from the lower surface. At this time, for example, a female screw is formed on an inner peripheral surface of the housing 20, a male screw is formed on the outer peripheral surface of the outlet 22, and these screws are screwed, whereby binding between the housing 20 and the outlet 22 can be strengthened.

<Positional relationship between inlet 21 and outlet 22>

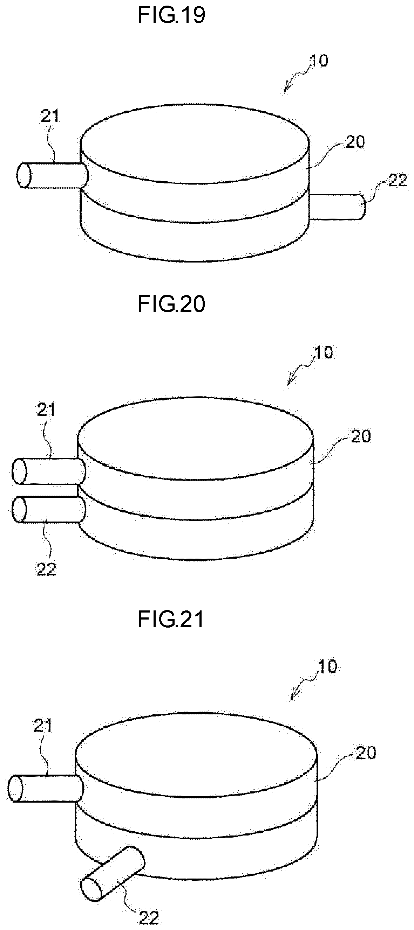

As illustrated in Fig. 19, the inlet 21 and the outlet 22 may be provided on a side surface of the columnar shape. In this case, since the inlet 21 and the outlet 22 need to be separated from each other by the concentration membrane 30, they need to be provided at different positions in the height direction of the columnar shape. As long as the inlet 21 and the outlet 22 are provided at such positions, the inlet 21 and the outlet 22 may be provided in opposite directions as illustrated in Fig. 19, may be provided in the same direction as illustrated in Fig. 20, or may be provided so as to be separated from each other at any plane angle as illustrated in Fig. 21.

<Internal shape of housing 20>

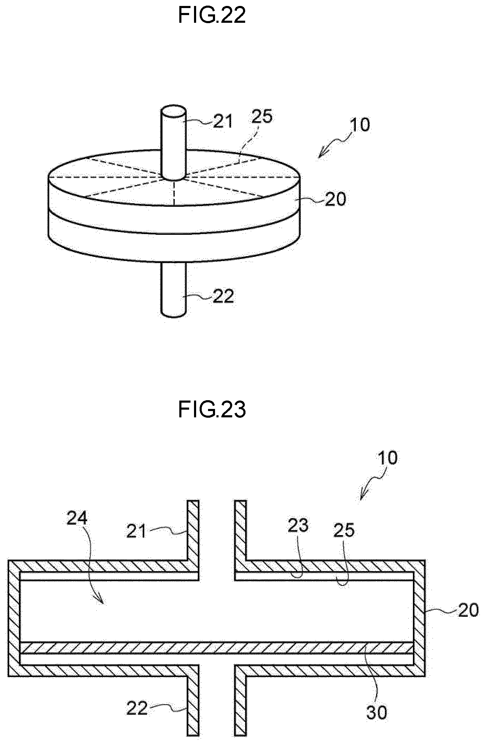

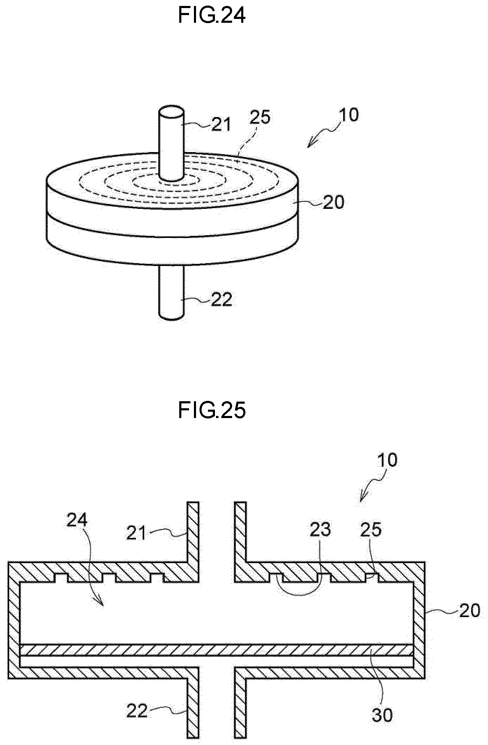

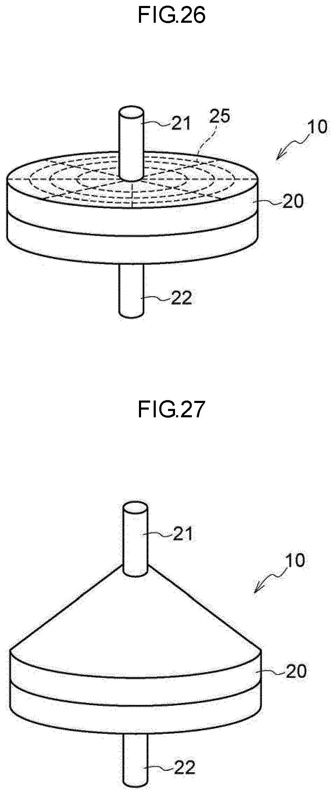

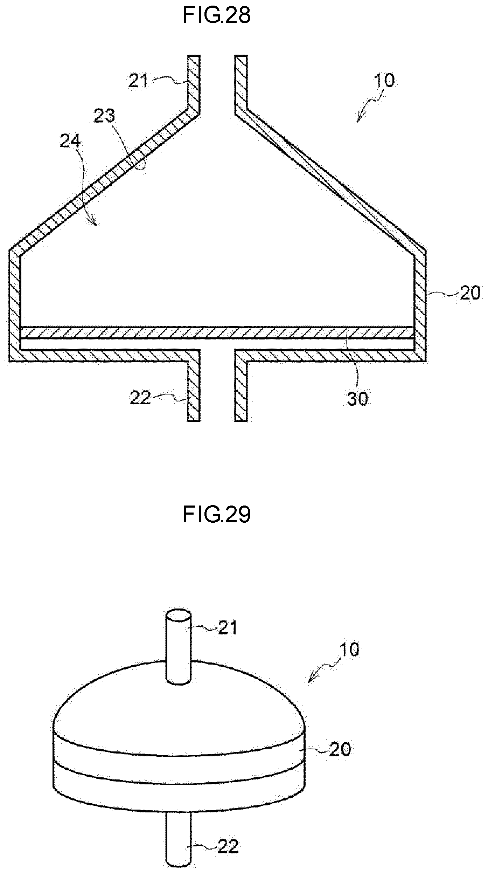

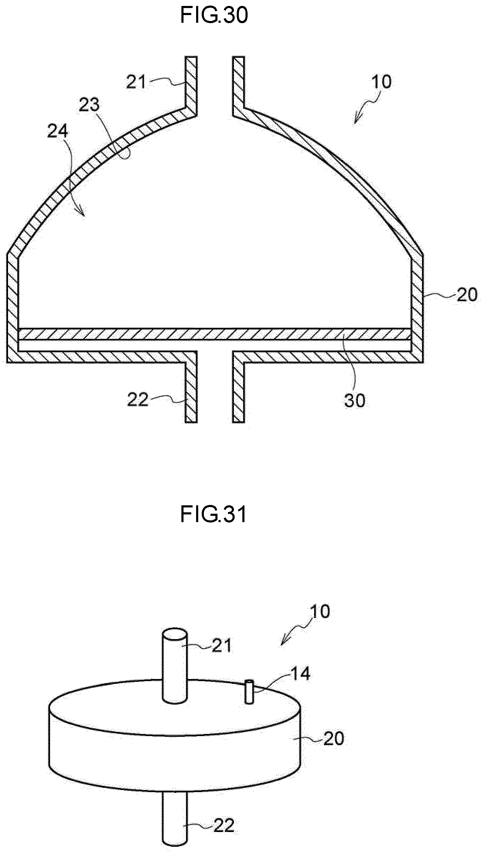

An inside of the housing 20 is not limited to the shape as illustrated in Figs. 1 and 2, and may have various shapes.
For example, in the housing 20, a guide groove 25 continuous from the inlet 21 may be formed in the inner wall portion 23 (see Fig. 2) facing the concentration space portion 24. For example, as illustrated in the perspective view of Fig. 22 and the cross-sectional view of Fig. 23, the guide groove 25 as a radial groove continuous from the inlet 21 can be provided in the inner wall portion 23 facing the concentration space portion 24 (in other words, an upper surface of the inner wall portion 23). In addition, as illustrated in the perspective view of Fig. 24 and the cross-sectional view of Fig. 25, the guide groove 25 as a spiral groove continuous from the inlet 21 can be provided in the inner wall portion 23 facing the concentration space portion 24. Further, as illustrated in a perspective view of Fig. 26, the guide groove 25 including a radial groove as illustrated in Fig. 22 and a groove that intersects with the radial groove and is concentric around the inlet 21 can be provided. By providing such a guide groove 25, the liquid to be treated 40 injected from the inlet 21 is easily guided to the concentration space portion 24 by a capillary force of the guide groove 25.
On the other hand, the housing 20 is formed in a tapered shape tapered toward the inlet 21 as illustrated in the perspective view of Fig. 27, so that, in the housing 20, the inner wall portion 23 facing the concentration space portion 24 can have a shape in which the diameter gradually increases from the inlet 21 toward the concentration membrane 30, as illustrated in the cross-sectional view of Fig. 28. In addition, the housing 20 is formed in a hemispherical shape protruding toward the inlet 21 as illustrated in the perspective view of Fig. 29, so that, in the housing 20, the inner wall portion 23 facing the concentration space portion 24 can have a shape in which the diameter gradually increases from the inlet 21 toward the concentration membrane 30, as illustrated in the cross-sectional view of Fig. 30. When the housing 20 has such a shape, the liquid to be treated 40 injected from the inlet can be guided to the concentration membrane 30 through an inclination of the inner wall portion 23. At this time, when the guide groove 25 as illustrated in Figs. 22 to 26 is provided in the inner wall portion 23, the liquid to be treated 40 can be more effectively guided.

<Method for recovering concentrated liquid 41>

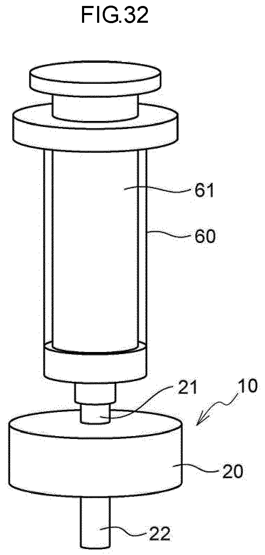

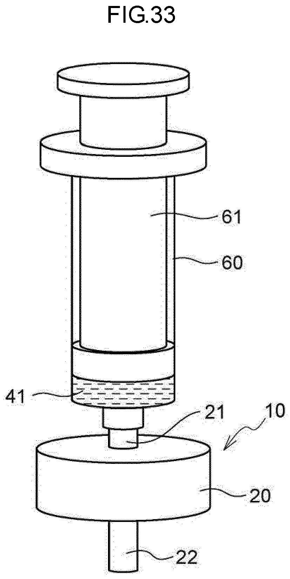

As illustrated in Fig. 6 described above, the concentrated liquid 41 can be recovered from the concentration space portion 24 by inserting a tip of an appropriate tool such as a micropipette from the inlet 21.
As illustrated in Fig. 31, a piece to be folded and removed 14 can be formed on an upstream side of the housing 20. When the fold piece 14 is folded and removed, a small hole appears on the upstream side of the housing 20. Then, after completion of the concentration of the liquid to be treated 40 by the concentration device 10, the tip of an appropriate tool such as a micropipette is inserted into the small hole, whereby the concentrated liquid 41 can be recovered from the concentration space portion 24.
Furthermore, after completion of the concentration of the liquid to be treated 40 by the concentration device 10, the syringe 60 is attached to the inlet 21 as illustrated in Fig. 32, and the plunger 61 is sucked, whereby the concentrated liquid 41 can be recovered into the syringe 60 as illustrated in Fig. 33.

<Concentration membrane>

As the concentration membrane 30, one having an appropriate material and an appropriate shape depending on the kind and properties of the biological particles 50 contained in the liquid to be treated 40 is used. When the biological particles 50 are, for example, particles formed of a lipid bilayer (for example, viruses, bacteria, or exosomes), the concentration membrane 30 desirably includes a hydrophilic composite porous membrane including a porous substrate and a hydrophilic resin with which at least one main surface and inner surfaces of pores of the porous substrate are coated. In the concentration membrane 30 of the present disclosure, "hydrophilic porous membrane onto which the biological particles are not adsorbed" means a porous membrane onto which the biological particles 50 are not adsorbed and which has hydrophilicity. The property of "hydrophilic porous membrane onto which the biological particles are not adsorbed" is not particularly limited because of balance with the properties of the target biological particles 50, but it can be said that such a porous membrane has hydrophilicity such that the biological particles 50 are not adsorbed since concentration is performed when the concentration rate exceeds 100% in a case where a concentration treatment is performed. For example, when the concentration membrane 30 contains a hydrophilic resin which will be described later or when a contact angle of water of the concentration membrane 30 is 90 degrees or less, it can be said that the concentration membrane 30 has "hydrophilicity", but the concentration membrane 30 in the present disclosure is not limited thereto.

The size of the biological particles 50 to be concentrated by the concentration membrane 30 of the present disclosure is not limited. A diameter or long axis length of the biological particles 50 is, for example, 1 nm or more, 5 nm or more, 10 nm or more, or 20 nm or more, and, for example, 100 μm or less, 50 μm or less, 1,000 nm or less, or 800 nm or less.

The concentration membrane 30 of the present disclosure may contain a member other than the hydrophilic composite porous membrane. Examples of the member other than the hydrophilic composite porous membrane include a sheet-like reinforcing member disposed in contact with a part or all of a main surface or a side surface of the hydrophilic composite porous membrane; and a guide member for mounting the concentration membrane 30 in the concentration device 10.

In the hydrophilic composite porous membrane included in the concentration membrane 30 of the present disclosure, at least the main surface on the upstream side during the concentration treatment may be coated with the hydrophilic resin, and both the main surfaces are preferably coated with the hydrophilic resin. Alternatively, the concentration membrane 30 may be a porous membrane having a monolayer structure containing a hydrophilic resin.

Examples of a coating form of the main surface of the porous substrate with the hydrophilic resin in the hydrophilic composite porous membrane include the coating forms of the main surface of the porous substrate described in <Concentration membrane> above, and the preferred coating form is also the same as that described therein.

Examples of a coating form of the inner surfaces of pores of the porous substrate with the hydrophilic resin in the hydrophilic composite porous membrane include the coating forms of the inner surfaces of pores of the porous substrate described in <Concentration membrane> above, and the preferred coating form is also the same as that described therein.

Concentration of the biological particles 50 using the concentration membrane 30 of the present disclosure is performed such that, when the liquid to be treated 40 is allowed to pass one main surface to the other main surface of the hydrophilic composite porous membrane, some or all of the biological particles 50 contained in the liquid to be treated 40 do not pass through the hydrophilic composite porous membrane but remain in the liquid to be treated 40 at at least any site of the upstream, the upstream-side main surface, and the inside of the pores of the hydrophilic composite porous membrane. A comparison is made between the liquid to be treated 40 before the concentration treatment and the liquid to be treated 40 recovered from at least any site of the upstream, the upstream-side main surface, and the inside of the pores of the hydrophilic composite porous membrane after the concentration treatment. When the concentration of the biological particles 50 contained in the latter liquid is higher, the biological particles 50 can be said to have been concentrated. A concentration rate (see the following formula) of the biological particles 50 achieved by the concentration membrane 30 of the present disclosure is more than 100%, preferably 200% or more, and more preferably 300% or more.

$$\text{Concentration rate } (\%) = (\text{biological particle concentration of liquid to be treated}$$
$$\text{recovered from at least any site of upstream, main surface on upstream side, and inside of}$$
$$\text{pores of hydrophilic composite porous membrane after concentration treatment}) \div (\text{biological}$$
$$\text{particle concentration of liquid to be treated before concentration treatment}) \times 100$$

Although the detailed mechanism is not necessarily clear, it is presumed that, when the hydrophilic composite porous membrane included in the concentration membrane 30 of the present disclosure has the hydrophilic resin on the upstream-side main surface and the inner surfaces of the pores, the biological particles 50 remaining at at least either of the upstream-side main surface and the inside of the pores of the hydrophilic composite porous membrane are easily recovered, and thus that the concentration rate of the biological particles 50 is improved.

The hydrophilic composite porous membrane, the porous substrate, and the hydrophilic resin in the concentration membrane 30 included in the concentration device 10 of the present disclosure are the same as the hydrophilic composite porous membrane, the porous substrate, and the hydrophilic resin in <Concentration membrane> above, and the form examples, the preferred forms, the physical properties, and the production methods are also the same as those of the hydrophilic composite porous membrane, the porous substrate, and the hydrophilic resin.

[Physical properties of concentration membrane 30]

In the concentration membrane 30, a contact angle of water as measured by the following measurement conditions is preferably 90 degrees or less on one side or both sides. The contact angle of water is preferably smaller. More preferably, the concentration membrane 30 is so hydrophilic that the contact angle of water, when attempted to be measured on one side or both sides under the following measurement conditions, cannot be measured because a water droplet penetrates into the membrane.

Here, the contact angle of water is a value as measured by the following measurement method. The concentration membrane 30 is left in an environment at a temperature of 25°C and a relative humidity of 60% for 24 hours or more to adjust the humidity. Thereafter, a water droplet of 1 $\mu$L of ion-exchanged water is dropped on the surface of the concentration membrane 30 with a syringe under an environment at the same temperature and the same humidity, and a contact angle 30 seconds after dropping of the water droplet is measured by a $\theta/2$ method using a fully automatic contact angle meter (Kyowa Interface Science Co., Ltd., model number: Drop Master DM 500).

The concentration membrane 30 used in the concentration device 10 of the present disclosure includes a hydrophilic composite porous membrane containing a porous substrate, and a hydrophilic resin with which at least one main surface and inner surfaces of pores of the porous substrate are coated, and that has a ratio t/x of a membrane thickness t ($\mu$m) to an average pore diameter x ($\mu$m), as measured with a perm porometer, is from 50 to 630.

When t/x of the concentration membrane 30 is less than 50, the membrane thickness t is too small for the average pore diameter x, or the average pore diameter x is too large for the membrane thickness t, and thus the biological particles 50 easily pass through the concentration membrane 30, so that a residual rate of the biological particles 50 remaining at at least any site of the upstream, the upstream-side main surface, and the inside of the pores of the concentration membrane 30 (hereinafter, simply referred to as "residual rate of the biological particles 50") is poor, and, as a result, the concentration rate of the biological particles 50 is poor. From this viewpoint, t/x is 50 or more, preferably 80 or more, and more preferably 100 or more.

When t/x of the concentration membrane 30 is more than 630, the membrane thickness t is too large for the average pore diameter x, or the average pore diameter x is too small for the membrane thickness t, and thus the liquid to be treated 40 is less likely to pass through the concentration membrane 30, and it takes time for the liquid to be treated 40 to pass through the concentration membrane 30 (that is, it takes time to concentrate the liquid to be treated 40). From this viewpoint, t/x is 630 or less, preferably 600 or less, more preferably 500 or less, further preferably 400 or less, and still further preferably 240 or less.

The thickness t of the concentration membrane 30 is preferably 10 $\mu$m or more, more preferably 15 $\mu$m or more, further preferably 20 $\mu$m or more, and still further preferably 30 $\mu$m or more from the viewpoint of increasing the strength of the concentration membrane 30 and the viewpoint of increasing the residual rate of the biological particles 50. The thickness t of the concentration membrane 30 is preferably 150 $\mu$m or less, more preferably 100 $\mu$m or less, further preferably 80 $\mu$m or less, and still further preferably 70 $\mu$m or less from the viewpoint of shortening a time necessary for the liquid to be treated 40 to pass through the concentration membrane 30 (hereinafter, referred to as treatment time for the liquid to be treated 40).

The thickness t of the concentration membrane 30 is determined by measuring values at 20 points with a contact type membrane thickness meter and averaging the measured values.

The average pore diameter x of the concentration membrane 30 as measured with a perm porometer is preferably 0.1 $\mu$m or more, more preferably 0.15 $\mu$m or more, and further preferably 0.2 $\mu$m or more, from the viewpoint of shortening the treatment time for the liquid to be treated 40 and the viewpoint of easily recovering the biological particles 50 remaining in the pores of the concentration membrane 30. The average pore diameter x of the concentration membrane 30 as measured with a perm porometer is preferably 0.5 $\mu$m or less, more preferably 0.45 $\mu$m or less, and further preferably 0.4 $\mu$m or less from the viewpoint of increasing the residual rate of the biological particles 50.

The average pore diameter x of the concentration membrane 30 as measured with a perm porometer is determined by a half dry method specified in ASTM E1294-89 using a perm porometer (PMI, model: CFP-1200 AEXL) and using Galwick (surface tension: 15.9 dyn/cm) manufactured by PMI as an immersion liquid. When only one main surface of the concentration membrane 30 is coated with the hydrophilic resin, the main surface coated with the hydrophilic resin is placed toward a pressurizing part of the perm porometer, and the measurement is performed.

A bubble point pore diameter y of the concentration membrane 30 as measured with a perm porometer is preferably more than 0.8 $\mu$m, more preferably 0.9 $\mu$m or more, and further preferably 1.0 $\mu$m or more, from the viewpoint of shortening the treatment time for the liquid to be treated 40 and the viewpoint of easily recovering the biological particles 50 remaining in the pores of the concentration membrane 30. The bubble point pore diameter y of the concentration membrane 30 as measured with a perm porometer is preferably 3 $\mu$m or less, more preferably 2.5 $\mu$m or less, and further preferably 2.2 $\mu$m or less from the viewpoint of increasing the residual rate of the biological particles 50.

The bubble point pore diameter y of the concentration membrane 30 as measured with a perm porometer is determined by a bubble point method (ASTM F316-86 and JIS K3832) using a perm porometer (PMI, model: CFP-1200 AEXL). However, the value is determined by changing the immersion liquid at the time of the test to Galwick (surface tension: 15.9 dyn/cm) manufactured by PMI. When only one main surface of the concentration membrane 30 is coated with the hydrophilic resin, the main surface coated with the hydrophilic resin is placed toward a pressurizing part of the perm porometer, and the measurement is performed.

A bubble point pressure of the concentration membrane 30 is, for example, 0.01 MPa or more and 0.20 MPa or less, and preferably 0.02 MPa to 0.15 MPa.

In the present disclosure, the bubble point pressure of the concentration membrane 30 is a value determined by immersing the polyolefin microporous membrane in ethanol, performing a bubble point test according to the bubble point test method of JIS K3832:1990, while changing the liquid temperature at the time of the test to $24 \pm 2°C$ and the applied pressure is increased at a pressure increase rate of 2 kPa/sec. When only one main surface of the concentration membrane 30 is coated with the hydrophilic resin, the main surface coated with the hydrophilic resin is placed toward a pressurizing part, and the measurement is performed.

A water flow rate f (mL/(min • $cm^2$ • MPa)) of the concentration membrane 30 is preferably 20 or more, more preferably 50 or more, and further preferably 100 or more from the viewpoint of shortening the treatment time for the liquid to be treated 40. The water flow rate f (mL/(min • $cm^2$ • MPa)) of the concentration membrane 30 is preferably 1,000 or less, more preferably 800 or less, and further preferably 700 or less from the viewpoint of increasing the residual rate of the biological particles 50.

The water flow rate f of the concentration membrane 30 is determined by allowing 100 mL of water to permeate a sample set on a liquid permeation cell having a constant liquid permeation area ($cm^2$) at a constant differential pressure (20 kPa), measuring a time (sec) necessary for 100 mL of water to permeate the sample, and subjecting the measured value to unit conversion. When only one main surface of the concentration membrane 30 is coated with the hydrophilic resin, water is allowed to permeate from the main surface coated with the hydrophilic resin to the main surface not coated with the hydrophilic resin, and the measurement is performed.

In the concentration membrane 30, the ratio f/y of the water flow rate f (mL/(min • $cm^2$ • MPa)) to the bubble point pore diameter y ($\mu$m) is preferably 100 or more, more preferably 150 or more, and further preferably 200 or more, from the viewpoint of shortening the treatment time for the liquid to be treated 40. In the concentration membrane 30, the ratio f/y of the water flow rate f (mL/(min • $cm^2$ • MPa)) to the bubble point pore diameter y ($\mu$m) is preferably 480 or less, more preferably 400 or less, and further preferably 350 or less, from the viewpoint of increasing the residual rate of the biological particles 50.

From the viewpoint of increasing a recovery rate of the biological particles 50, the concentration membrane 30 has a surface roughness Ra of preferably 0.3 $\mu$m or more, and more preferably 0.4 $\mu$m or more, at least on the main surface on the upstream side during the concentration treatment. From the viewpoint of increasing a recovery rate of the biological particles 50, the concentration membrane 30 has a surface roughness Ra of preferably 0.7 $\mu$m or less, and more preferably 0.6 $\mu$m or less, at least on the main surface on the upstream side during the concentration treatment.

The surface roughness Ra of the concentration membrane 30 is determined by measuring surface roughnesses at three random places on the surface of a sample in a non-contact manner using a light wave interference type surface roughness meter (Zygo Corporation, NewView 5032), and using analysis software (optional application: Advance Texture.app) for roughness evaluation.

A Gurley value (seconds/100 mL • $\mu$m) per unit thickness of the concentration membrane 30 is, for example, 0.001 to 5, preferably 0.01 to 3, and more preferably 0.05 to 1. The Gurley value of the concentration membrane 30 is a value as measured according to JIS P8117:2009.

A porosity of the concentration membrane 30 is, for example, 70% to 90%, preferably 72% to 89%, and more preferably 74% to 87%. The porosity of the concentration membrane 30 is determined according to the following calculation method. That is, regarding constituent material 1, constituent material 2, constituent materials 3,..., and constituent material n of the concentration membrane 30, when masses of the respective constituent materials are $W_1$, $W_2$, $W_3$,..., and $W_n$ (g/$cm^2$), true densities of the constituent materials are $d_1$, $d_2$, $d_3$,..., and $d_n$ (g/$cm^3$), and the membrane thickness is t (cm), the porosity $\varepsilon$ (%) is determined according to the following formula.

$$\varepsilon = \left( 1 - \frac{\sum_{i=1}^{n} \frac{Wi}{di}}{t} \right) \times 100$$

The concentration membrane 30 is preferably less likely to curl from the viewpoint of handleability. From the viewpoint of suppressing curling of the concentration membrane 30, both the main surfaces of the concentration membrane 30 are preferably coated with the hydrophilic resin.

The concentration device 10 of the present disclosure uses the concentration membrane 30 as described above, and thus can concentrate the biological particles 50 more easily and more rapidly than the centrifugal separation method. By using the concentration membrane 30 of the present disclosure, the biological particles 50 can be concentrated more rapidly and efficiently as compared with when conventional porous membranes are used.

[Shape of concentration membrane 30]

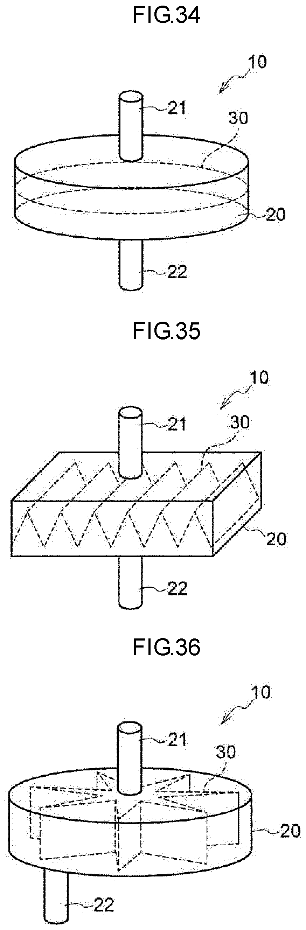

A shape of the concentration membrane 30 in the housing 20 can be flat as illustrated in Fig. 34. In addition, the concentration membrane 30 may be folded in one direction, as illustrated in Fig. 35. Further, the concentration membrane 30 may be folded in the circumferential direction, as illustrated in Fig. 36.

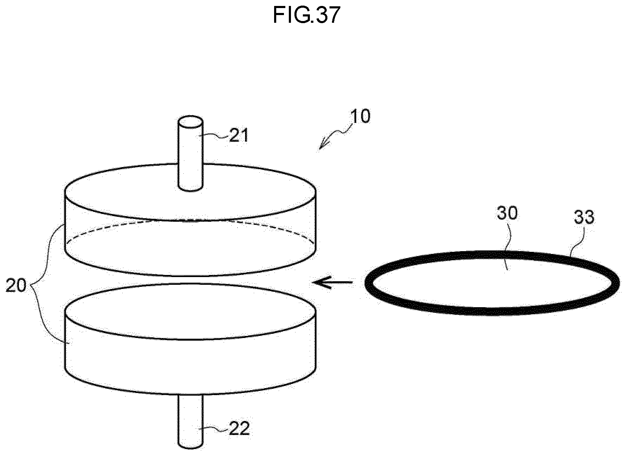

In addition, as illustrated in Fig. 37, an annular frame member 33 may be attached to a peripheral edge of the circular concentration membrane 30, and the concentration membrane 30 may be detachably attached in the housing 20 divided into two in the vertical direction.

<Concentration system 70 for biological particles 50>

A concentration system 70 for the biological particles 50 is configured by combining any of the concentration devices 10 for the biological particles 50 described above with a unit for applying a differential pressure between the inlet 21 and the outlet 22.

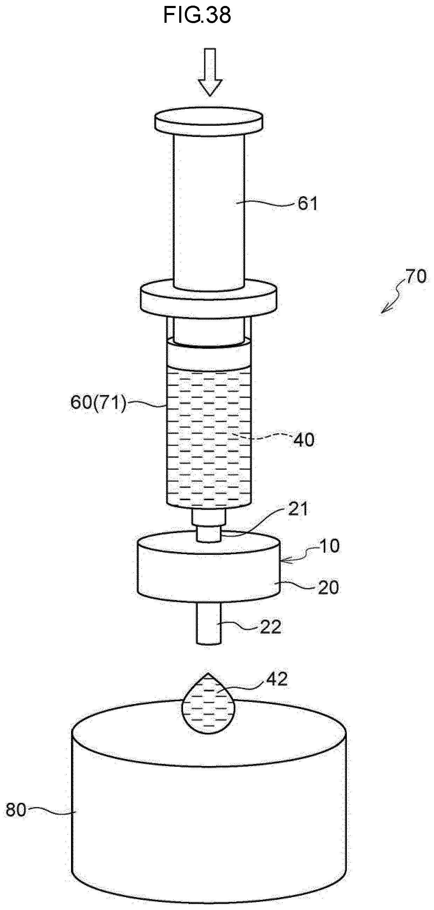

For example, in an example illustrated in Fig. 38, the syringe 60 as a pressurization unit 71 is attached to the inlet 21 of the concentration device 10. It is desirable to reliably connect the inlet 21 and the syringe 60 by, for example, a luer lock (see Fig. 13). The liquid to be treated 40 is stored in the syringe 60. On the other hand, a waste liquid tank 80 is installed below the outlet 22 of the concentration device 10. The concentration device 10 is supported by an appropriate device such as a stand (not illustrated) together with the syringe. From this state, when the plunger 61 is pressed manually or by an appropriate device in the pressurization unit 71, the liquid to be treated 40 is pressurized, passes through the concentration membrane 30 in the housing 20, and falls, as the effluent 42, from the outlet 22 to the waste liquid tank 80 installed below.

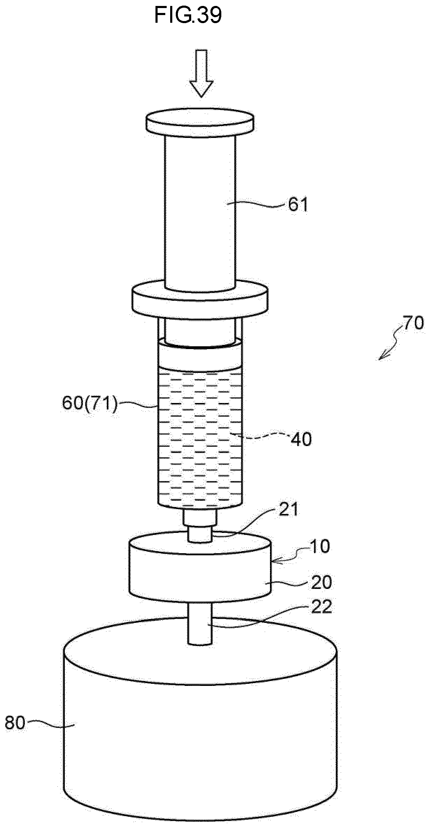

In an example illustrated in Fig. 39, the syringe 60 as the pressurization unit 71 is attached to the inlet 21 of the concentration device 10. It is desirable to reliably connect the inlet 21 and the syringe 60 by, for example, a luer lock (see Fig. 13). The liquid to be treated 40 is stored in the syringe 60. On the other hand, the waste liquid tank 80 is connected to the outlet 22 of the concentration device 10. It is desirable also to reliably connect the outlet 22 and the waste liquid tank 80 by, for example, a luer lock (see Fig. 17). In this example, the concentration device 10 is self-supported by the waste liquid tank 80 together with the syringe 60. From this state, when the plunger 61 is pressed manually or by an appropriate device in the pressurization unit 71, the liquid to be treated 40 is pressurized, passes through the concentration membrane 30 in the housing 20, and flows, as the effluent 42, from the outlet 22 into the waste liquid tank 80 installed below. Here, since a portion from the outlet 22 to the waste liquid tank 80 is hermetically sealed with respect to the outside world, scattering of the effluent 42 which has fallen into the waste liquid tank 80 to the surroundings is prevented.

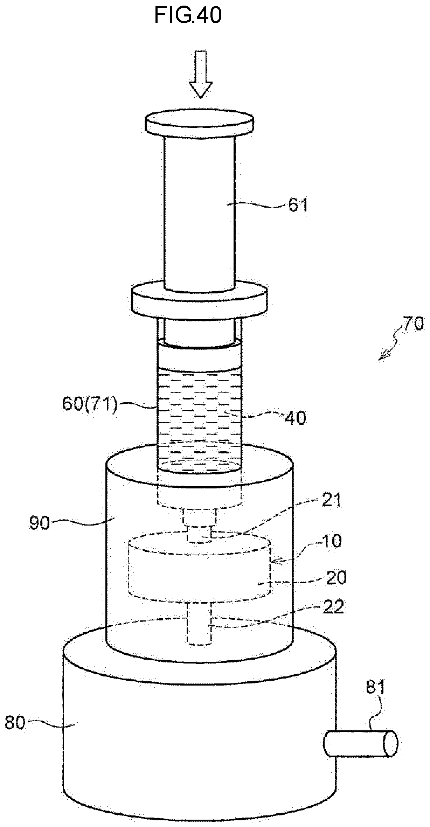

Furthermore, in an example illustrated in Fig. 40, the syringe 60 as the pressurization unit 71 is attached to the inlet 21 of the concentration device 10. It is desirable to reliably connect the inlet 21 and the syringe 60 by, for example, a luer lock (see Fig. 13). The liquid to be treated 40 is stored in the syringe 60. On the other hand, the waste liquid tank 80 is connected to the outlet 22 of the concentration device 10. It is desirable also to reliably connect the outlet 22 and the waste liquid tank 80 by, for example, a luer lock (see Fig. 17). Furthermore, the entire concentration device 10 is covered with a cylindrical guard unit 90 in order to prevent scattering of the liquid to be treated 40. From a tip portion of the syringe 60, an exhaust port 81 for removing air from the inside is opened in the waste liquid tank 80 of the waste liquid tank 80 including the concentration device 10. An infection prevention filter (not illustrated) is attached in the middle of the exhaust port 81. From this state, when the plunger 61 is pressed manually or by an appropriate device in the pressurization unit 71, the liquid to be treated 40 is pressurized, passes through the concentration membrane 30 in the housing 20, and flows, as the effluent 42, from the outlet 22 into the waste liquid tank 80 installed below. Here, since a portion from the outlet 22 to the waste liquid tank 80 is hermetically sealed with respect to the outside world, contamination of the surroundings by scattering of the effluent 42 which has fallen into the waste liquid tank 80 is prevented. In addition, since the infection prevention filter is attached to the exhaust port 81, scattering of the biological particles 50, which have passed through the concentration membrane 30 and fallen into the waste liquid tank 80 together with the effluent 42, to the surroundings is prevented. A depressurization unit 72 which will be described later may be coupled to the exhaust port 81 to simultaneously perform pressing by the pressurization unit 71 and suction by the depressurization unit 72.

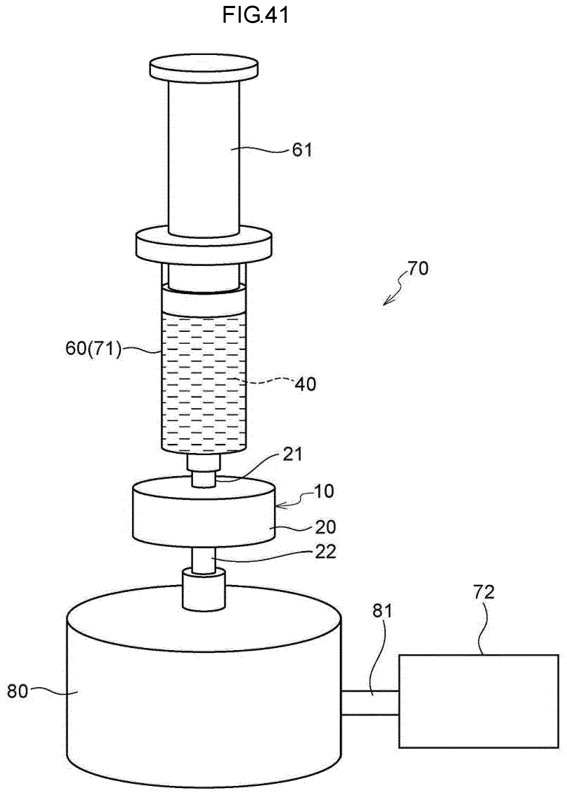

In addition, in an example illustrated in Fig. 41, the syringe 60 for storing the liquid to be treated 40 is attached to the inlet 21 of the concentration device 10. It is desirable to reliably connect the inlet 21 and the syringe 60 by, for example, a luer lock (see Fig. 13). On the other hand, the waste liquid tank 80 is connected to the outlet 22 of the concentration device 10. It is desirable also to reliably connect the outlet 22 and the waste liquid tank 80 by, for example, a luer lock (see Fig. 17). The exhaust port 81 for removing air from the inside is opened in the waste liquid tank 80. An infection prevention filter (not illustrated) is attached in the middle of the exhaust port 81. The depressurization unit 72 is coupled to the exhaust port 81. From this state, when the depressurization unit 72 is operated to suck the air in the waste liquid tank 80, the inside of the housing 20 is depressurized. Then, the liquid to be treated 40 in the syringe 60 is sucked into the housing 20, passes through the concentration membrane 30, and flows out, as the effluent 42, from the outlet 22 to the waste liquid tank 80 installed below. Here, since a portion from the outlet 22 to the waste liquid tank 80 is hermetically sealed with respect to the outside world, contamination of the surroundings by scattering of the effluent 42 which has fallen into the waste liquid tank 80 is prevented. In addition, since the infection prevention filter is attached to the exhaust port 81, contamination of the depressurization unit 72 by the biological particles 50 that have passed through the concentration membrane 30 and fallen into the waste liquid tank 80 together with the effluent 42 is prevented.

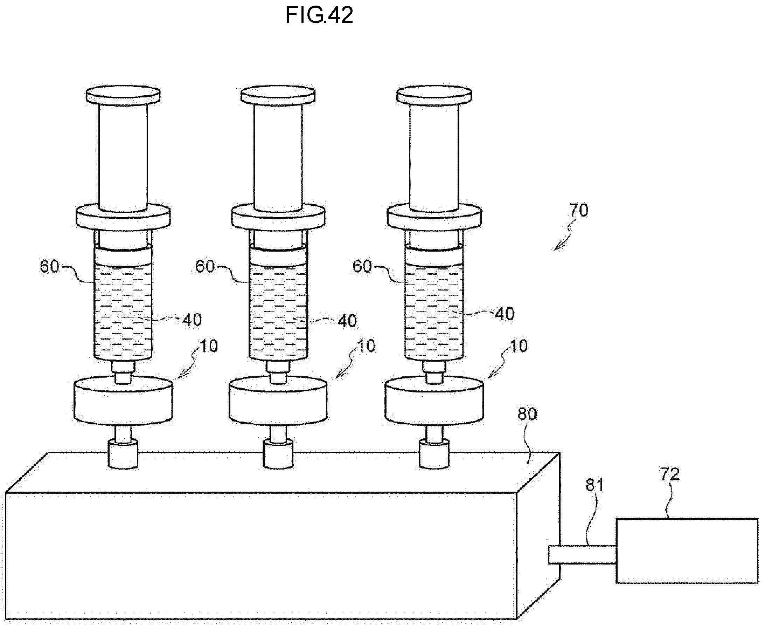

Note that, as in an example illustrated in Fig. 42, it is also possible to install a plurality of sets of the concentration devices 10 to which the syringes 60 are attached as illustrated in Fig. 41 in one waste liquid tank 80 and to treat a plurality of specimens of the liquid to be treated 40 by one depressurization unit 72. The exhaust port 81 and the depressurization unit 72 are also similar to those of the example illustrated in Fig. 41.

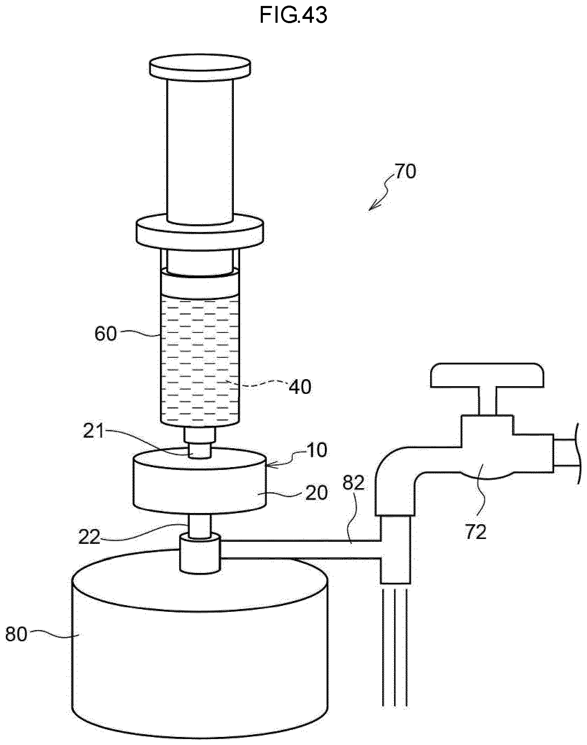

Furthermore, in an example illustrated in Fig. 43, the syringe 60 for storing the liquid to be treated 40 is attached to the inlet 21 of the concentration device 10. It is desirable to reliably connect the inlet 21 and the syringe 60 by, for example, a luer lock (see Fig. 13). On the other hand, the waste liquid tank 80 is connected to the outlet 22 of the concentration device 10. It is desirable also to reliably connect the outlet 22 and the waste liquid tank 80 by, for example, a luer lock (see Fig. 17). Furthermore, in the present example, a suction port 82 branches from the outlet 22, and the depressurization unit 72 by a tap is connected to a tip of the suction port 82. Note that an infection prevention filter (not illustrated) is attached in the middle of the suction port 82. When the tap as the depressurization unit 72 is opened from this state, the air in the housing 20 is sucked toward the suction port 82 by water flow, whereby the inside of the housing 20 is depressurized. Then, the liquid to be treated 40 in the syringe 60 is sucked into the housing 20, passes through the concentration membrane 30, and flows out, as the effluent 42, from the outlet 22 to the waste liquid tank 80 installed below. Here, since a portion from the outlet 22 to the waste liquid tank 80 is hermetically sealed with respect to the outside world, contamination of the surroundings by scattering of the effluent 42 which has fallen into the waste liquid tank 80 is prevented. In addition, since the infection prevention filter is attached to the suction port 82, contamination of the water flow by the biological particles 50 that have passed through the concentration membrane 30 and fallen into the waste liquid tank 80 together with the effluent 42 is prevented.

EXAMPLES

Hereinafter, the concentration membrane and the concentration device of the present disclosure will be described more specifically with reference to Examples.

Materials, amounts used, proportions, treatment procedures, and the like presented in the following Examples can be appropriately changed without departing from the gist of the present disclosure. Therefore, the scope of the concentration membrane and the concentration device of the present disclosure should not be construed as being limited by the specific examples which will be described below.

<Preparation of hydrophilic composite porous membrane>

[Example 1 (Sample 1)]

-Preparation of polyethylene microporous membrane-

A polyethylene composition was prepared by mixing 3.75 parts by mass of ultra-high molecular weight polyethylene (hereinafter referred to as "UHMWPE") having a weight average molecular weight of 4.6 million with 21.25 parts by mass of high-density polyethylene (hereinafter referred to as "HDPE") having a weight average molecular weight of 560,000 and a density of 950 kg/m$^3$. The polyethylene composition and decalin were mixed so that the polymer concentration was 25% by mass to prepare a polyethylene solution.

The polyethylene solution was extruded from a die at a temperature of 147°C into a sheet, and then the extrudate was cooled in a water bath at a water temperature of 20°C to obtain a first gel-like sheet.

The first gel-like sheet was preliminarily dried in a temperature atmosphere at 70°C for 10 minutes, then subjected to primary stretching at 1.8 times in the MD direction, and then subjected to main drying in a temperature atmosphere at 57°C for 5 minutes to obtain a second gel-like sheet (base tape) (an amount of the solvent remaining in the second gel-like sheet was less than 1%). Next, as secondary stretching, the second gel-like sheet (base tape) was stretched at a magnification of 4 times at a temperature of 90°C in the MD direction, subsequently stretched at a magnification of 9 times at a temperature of 125°C in the TD direction, and then immediately subjected to a heat treatment (heat fixation) at 144°C.

The decalin in the sheet was extracted while the heat-fixed sheet was continuously immersed for 30 seconds in each of two tanks into which a methylene chloride bath was divided. After the sheet was conveyed from the methylene chloride bath, methylene chloride was removed by drying in a temperature atmosphere at 40°C. In this way, a polyethylene microporous membrane was obtained.

-Hydrophilization treatment for polyethylene microporous membrane-

As a hydrophilic resin, an ethylene/vinyl alcohol binary copolymer (Soarnol DC 3203R manufactured by The Nippon Synthetic Chemical Industry Co., Ltd., ethylene unit: 32% by mol (hereinafter, referred to as EVOH)) was prepared. The EVOH was dissolved in a mixed solvent of 1-propanol and water (1-propanol:water = 3:2 [volume ratio]) so that the concentration of the EVOH was 0.2% by mass, to obtain a coating liquid.

The polyethylene microporous membrane fixed to a metal frame was immersed in the coating liquid to impregnate the pores of the polyethylene microporous membrane with the coating liquid, and then the polyethylene microporous membrane was pulled up. Next, an excess coating liquid adhering to both main surfaces of the polyethylene microporous membrane was removed, and the membrane was dried at normal temperature for 2 hours. Then, the metal frame was removed from the polyethylene microporous membrane. In this way, a hydrophilic composite porous membrane in which both the main surfaces and inner surfaces of pores of the polyethylene microporous membrane were coated with the hydrophilic resin was obtained.

[Examples 2 to 7 (Samples 2 to 7)]

-Preparation of polyethylene microporous membrane-

A polyethylene microporous membrane was produced in the same manner as in Example 1 (Sample 1) except that the composition of the polyethylene solution or the production step for the polyethylene microporous membrane was changed as shown in Table 1. In Examples 3 to 6 (Samples 3 to 6), after the sheet was conveyed from the methylene chloride bath, methylene chloride was removed by drying in a temperature atmosphere at 40°C, and an annealing treatment was performed while the sheet was conveyed on a roller heated to 120°C.

-Hydrophilization treatment for polyethylene microporous membrane-

In the same manner as in Example 1 (Sample 1), EVOH was applied to the polyethylene microporous membrane to prepare a hydrophilic composite porous membrane. However, in Examples 5 and 6 (Samples 5 and 6), the EVOH concentration of the coating liquid was 1% by mass.

[Comparative Example 1 (Sample 8)]

-Preparation of polyethylene microporous membrane-

A polyethylene microporous membrane was produced in the same manner as in Example 1 (Sample 1) except that the composition of the polyethylene solution and the production step for the polyethylene microporous membrane were changed as shown in Table 1. In Comparative Example 1 (Sample 8), after the sheet was conveyed from the methylene chloride bath, methylene chloride was removed by drying in a temperature atmosphere at 40°C, and an annealing treatment was performed while the sheet was conveyed on a roller heated to 120°C.

-Hydrophilization treatment for polyethylene microporous membrane-

One side of the polyethylene microporous membrane was subjected to a plasma treatment (AP-300 manufactured by Nordson MARCH, output: 150W, treatment pressure: 400 mTorr, gas flow rate: 160 sccm, treatment time: 45 seconds) to obtain a hydrophilic composite porous membrane.

[Comparative Example 2 (Sample 9)]

-Preparation of polyethylene microporous membrane-

A polyethylene microporous membrane was produced in the same manner as in Example 1 (Sample 1) except that the production step for the polyethylene microporous membrane was changed as shown in Table 1.

-Hydrophilization treatment for polyethylene microporous membrane-

One side of the polyethylene microporous membrane was subjected to a plasma treatment (AP-300 manufactured by Nordson MARCH, output: 150W, treatment pressure: 400 mTorr, gas flow rate: 160 sccm, treatment time: 45 seconds) to obtain a hydrophilic composite porous membrane.

[Comparative Example 3 (Sample 10)]

-Preparation of polyethylene microporous membrane-

A polyethylene microporous membrane was produced in the same manner as in Example 1 (Sample 1) except that the production step for the polyethylene microporous membrane was changed as shown in Table 1.

-Hydrophilization treatment for polyethylene microporous membrane-

In the same manner as in Example 1 (Sample 1), EVOH was applied to the polyethylene microporous membrane to prepare a hydrophilic composite porous membrane.

[Comparative Example 4 (Sample 11)]

As Comparative Example 4 (Sample 11), SYNN0601MNXX104 manufactured by MDI Corporation as a syringe filter was prepared. A porous membrane included in the syringe filter is made of nylon.

[Comparative Example 5 (Sample 12)]

As Comparative Example 5 (Sample 12), CA025022 manufactured by Membrane Solutions Co., Ltd. as a syringe filter was prepared. A porous membrane included in the syringe filter is made of cellulose acetate.

<Measurement of physical properties of hydrophilic composite porous membrane>

Using each of the hydrophilic composite porous membranes of Examples 1 to 7 (Samples 1 to 7) and Comparative Examples 1 to 5 (Samples 8 to 12) or a porous membrane as a sample, the following physical properties were measured. For each of the hydrophilic composite porous membranes of Comparative Examples 1 and 2 (Samples 8 and 9), the physical properties of the plasma-treated main surface were measured. For each of porous membranes included in the syringe filters of Comparative Examples 4 and 5 (Samples 11 and 12), the porous membrane was taken out from the syringe filter, and the physical properties of the main surface on a syringe filter inlet side were measured. The results are shown in Table 2.

[Membrane thickness]

The membrane thickness of the hydrophilic composite porous membrane or the porous membrane were determined by measuring values at 20 points with a contact type membrane thickness meter (manufactured by Mitutoyo Corporation), and averaging the measured values. As a contact terminal, a columnar terminal having a bottom surface with a diameter of 0.5 cm was used. A measurement pressure was 0.1N.

[Average pore diameter x]

The average pore diameter x ($\mu$m) of the hydrophilic composite porous membrane or the porous membrane was determined by a half dry method specified in ASTM E1294-89 using a perm porometer (model: CFP-1200 AEXL) manufactured by PMI and using Galwick (surface tension: 15.9 dyn/cm) manufactured by PMI as an immersion liquid. A measurement temperature was 25°C, and a measurement pressure was changed in a range of 0 to 600 kPa.

[Bubble point pore diameter y]

The bubble point pore diameter y ($\mu$m) of the hydrophilic composite porous membrane or the porous membrane was determined by a bubble point method (defined in ASTM F316-86 and JIS K3832:1990) using a perm porometer (model: CFP-1200 AEXL) manufactured by PMI. However, the value is determined by changing the immersion liquid at the time of the test to Galwick (surface tension: 15.9 dyn/cm) manufactured by PMI. A measurement temperature was 25°C, and a measurement pressure was changed in a range of 0 to 600 kPa.

[Bubble point pressure]

The bubble point pressure of the hydrophilic composite porous membrane or the porous membrane is a value determined by immersing the hydrophilic composite porous membrane or the porous membrane in ethanol, and performing a bubble point test according to a bubble point test method of JIS K3832:1990, provided that a liquid temperature at the time of the test is changed to 24 $\pm$ 2°C, and that the applied pressure is increased at a pressure increase rate of 2 kPa/sec.

[Water flow rate f]

The hydrophilic composite porous membrane was cut out into a size of 10 cm in the MD direction $\times$ 10 cm in the TD direction, and set on a stainless steel circular liquid permeation cell having a liquid permeation area of 17.34 cm$^2$ • One hundred (100) mL of water was allowed to permeate at a differential pressure of 20 kPa, and a time (sec) necessary for 100 mL of water to permeate was measured. The measurement was performed in a temperature atmosphere at a room temperature of 24°C. The water flow rate f (mL/(min • cm$^2$ • MPa)) was determined by subjecting the measurement conditions and the measured value to unit conversion.

[Surface roughness Ra]

An arithmetic average height under the following conditions was measured using a light wave interference type surface roughness meter (Zygo Corporation, NewView 5032) to determine the surface roughness Ra.

- Objective lens: 20 $\times$ Mirau type
- Image zoom: 1.0 $\times$
- FDA Res: Normal or Low
- Analysis conditions: After obtainment of data on three places of each sample in a non-contact manner using Stich.app, which is a standard application of Zygo Corporation, the surface roughness was analyzed using Advance Texture.app, which is an optional application for roughness evaluation.

<Evaluation of performance of concentration membrane>

A concentration test was performed using each of the hydrophilic composite porous membranes of Examples 1 to 7 (Samples 1 to 7) and Comparative Examples 1 to 5 (Samples 8 to 12) or the porous membrane as a concentration membrane. When each of the hydrophilic composite porous membranes of Comparative Examples 1 and 2 (Samples 8 and 9) was used as a concentration membrane, the plasma-treated main surface was set to the upstream side. When each of the porous membranes included in the syringe filters of Comparative Example 4 and 5 (Samples 11 and 12) was used as a concentration membrane, the porous membrane was taken out from the syringe filter, and the main surface on the syringe filter inlet side was set to the upstream side. The concentration test results are shown in Table 2. Details of the concentration test are as follows.

A virus suspension in which dengue fever viruses were suspended in a buffer solution was prepared. A viral unit was 1 $\times$ 10$^4$ FFU/mL. The dengue viruses are spherical viruses having an envelope and a diameter of about 40 nm to about 60 nm.

The hydrophilic composite porous membrane or porous membrane was punched into a circle having a diameter of 13 mm with a punch, and installed in a housing of a filter holder (Swinnex 35 manufactured by Merck Millipore) to prepare a concentration device. The housing is provided with an inlet and an outlet, and, in the housing, a concentration space portion is provided on an upstream side of the hydrophilic composite porous membrane or the porous membrane used as the concentration membrane (see Fig. 2). Ten (10) mL of the virus suspension was collected in a 10 mL-volume syringe (manufactured by Terumo Corporation). Then, as in the example illustrated in Fig. 38, a tip of the syringe was connected to the concentration device, and the virus suspension was allowed to pass through the concentration device. A pressure applied to a plunger was about 30 N. When the plunger was not moved by the pressure, the applied pressure was gradually increased to apply the minimum pressure at which the plunger was moved.

[Treatment time]

A time (seconds) from a time when the plunger was started to be pushed to a time when the plunger was fully pushed was measured.

[Concentration rate]

After the plunger was fully pushed, the plunger was reciprocated several times in a state where the concentration device faced up and the syringe faced down, and the virus suspension remaining upstream of the concentration membrane was recovered. The recovered virus suspension was used as a sample, and the total RNA was extracted by using Viral RNA Mini Kit (manufactured by QIAGEN). The extracted total RNA was reversetranscripted by using ReverTra Ace (registered trade name, manufactured by TOYOBO CO., LTD) to produce cDNA. The virus RNA in the sample was quantified by performing qRT-PCR by using a primer which specifically binds to the RNA of dengue fever virus, and by using SYBR Green I (SYBR is a registered trade name, manufactured by TAKARA BIO INK.). Concentration rate (%) = $Cb \div Ca \times 100$ was calculated from a concentration Ca of the virus RNA concentration in the virus suspension before liquid flow and a concentration Cb of the virus RNA concentration in the recovered virus suspension.

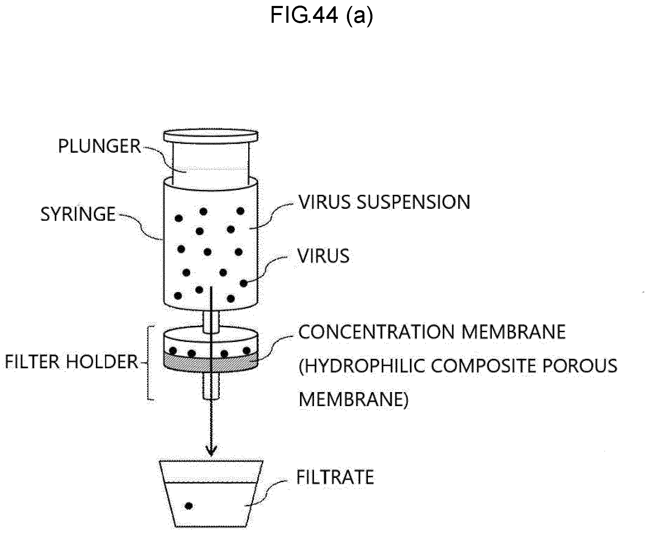

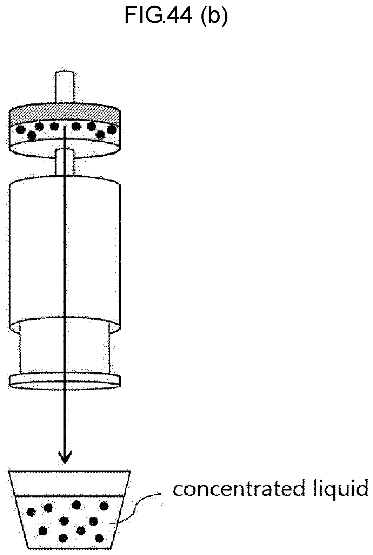

Fig. 44 is a schematic diagram showing an instrument and an operation for the concentration test. An arrow in Fig. 44(a) indicates a direction in which the virus suspension flows. An arrow in Fig. 44(b) indicates a direction in which the virus suspension remaining upstream of the concentration membrane is recovered.

[Table 1]

| | | | Example 1 | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|---|---|
| | Sample No. | | 1 | 2 | 3 | 4 | 5 | 6 | 7 | 8 | 9 | 10 |
| Composition of polyethylene solution | Decalin | Parts by mass | 75 | 75 | 75 | 75 | 75 | 75 | 75 | 75 | 75 | 75 |
| | UHMWPE | Mw | 4.6 million | 4.6 million | 4.6 million | 4.6 million | 4.6 million | 4.6 million | 4.6 million | 4.6 million | 4.6 million | 4.6 million |
| | | Parts by mass | 3.75 | 3.75 | 7.5 | 5 | 7.5 | 7.5 | 5 | 7.5 | 3.75 | 3.75 |
| | HDPE | Mw | 560,000 | 560,000 | 560,000 | 560,000 | 560,000 | 560,000 | 560,000 | 560,000 | 560,000 | 560,000 |
| | | Parts by mass | 21.25 | 21.25 | 17.5 | 20 | 17.5 | 17.5 | 20 | 17.5 | 21.25 | 21.25 |
| | Polymer concentration | % by mass | 25 | 25 | 25 | 25 | 25 | 25 | 25 | 25 | 25 | 25 |
| Extrusion | Die temperature | °C | 147 | 149 | 148 | 148 | 149 | 148 | 148 | 148 | 147 | 148 |
| | Cooling temperature | °C | 20 | 20 | 20 | 20 | 20 | 20 | 20 | 20 | 20 | 20 |
| | Preliminary drying temperature | °C | 70 | 70 | 70 | 70 | 70 | 70 | 70 | 70 | 70 | 70 |
| | Preliminary drying time | Minutes | 10 | 10 | 10 | 10 | 10 | 10 | 10 | 10 | 10 | 10 |
| | Primary stretching ratio | Times | 1.8 | 1.8 | 1.1 | 1.3 | 1.1 | 1.6 | 1.4 | 1.6 | 1.5 | 1.4 |
| | Main drying temperature | °C | 57 | 57 | 57 | 57 | 57 | 57 | 57 | 57 | 57 | 57 |
| | Main drying time | Minutes | 5 | 5 | 5 | 5 | 5 | 5 | 5 | 5 | 5 | 5 |

(continued)

| | | | Example 1 | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|---|---|
| | Sample No. | | 1 | 2 | 3 | 4 | 5 | 6 | 7 | 8 | 9 | 10 |
| Stretching | MD stretching temperature | °C | 90 | 90 | 90 | 90 | 90 | 90 | 90 | 90 | 90 | 90 |
| | MD stretching ratio | Times | 4 | 4 | 2 | 2 | 6.5 | 4.5 | 3.6 | 4.5 | 3 | 3 |
| | TD stretching temperature | °C | 125 | 103 | 130 | 130 | 130 | 125 | 125 | 125 | 125 | 125 |
| | TD stretching ratio | Times | 9 | 9 | 5 | 5 | 13.5 | 9 | 9 | 9 | 9 | 9 |
| | Heat fixation temperature | °C | 144 | 120 | 140 | 140 | 142 | 147 | 144 | 147 | 140 | 144 |
| Extraction | Extraction time | Seconds | 60 | 60 | 60 | 60 | 60 | 60 | 60 | 60 | 60 | 60 |
| | Drying temperature | °C | 40 | 40 | 40 | 40 | 40 | 40 | 40 | 40 | 40 | 40 |
| | Annealing temperature | °C | - | - | 120 | 120 | 120 | 120 | - | 120 | - | - |
| Hydrophilization treatment | EVOH coating | | Coated | Coated | Coated | Coated | Coated | Coated | Coated | - | - | Coated |
| | Plasma treatment | | - | - | - | - | - | - | - | One side | One side | - |

[Table 2]

| | Sample No. | Hydrophilization treatment | Membrane thickness t | Average pore diameter x | t/x | BP pressure | BP pore diameter y | Water flow rate at differential pressure of 20 kPa | Water flow rate f | f/y | Surface roughness Ra | Treatment time | Concentration rate |
|---|---|---|---|---|---|---|---|---|---|---|---|---|---|
| | | | μm | μm | - | MPa | μm | mL/min·cm² | mL/min·cm²·MPa | - | μm | sec. | % |
| Example 1 | 1 | EVOH coating | 54 | 0.24 | 226 | 0.05 | 0.93 | 6.8 | 342 | 367 | 0.42 | 40 | 931 |
| Example 2 | 2 | EVOH coating | 58 | 0.37 | 156 | 0.02 | 2.18 | 12.0 | 600 | 275 | 0.60 | 34 | 652 |
| Example 3 | 3 | EVOH coating | 46 | 0.19 | 241 | 0.09 | 0.61 | 4.7 | 235 | 386 | 0.40 | 72 | 336 |
| Example 4 | 4 | EVOH coating | 52 | 0.45 | 115 | 0.06 | 1.10 | 15.0 | 750 | 682 | 0.45 | 26 | 470 |
| Example 5 | 5 | EVOH coating | 19 | 0.11 | 181 | 0.18 | 0.27 | 0.9 | 43 | 163 | 0.34 | 60 | 156 |
| Example 6 | 6 | EVOH coating | 34 | 0.15 | 230 | 0.12 | 0.52 | 2.5 | 124 | 239 | 0.48 | 71 | 709 |
| Example 7 | 7 | EVOH coating | 61 | 0.13 | 470 | 0.12 | 0.45 | 1.9 | 96 | 212 | 0.35 | 96 | 239 |
| Comparative Example 1 | 8 | Plasma treatment | 35 | 0.36 | 96 | 0.08 | 0.87 | 9.6 | 478 | 549 | 0.57 | 47 | 89 |
| Comparative Example 2 | 9 | Plasma treatment | 49 | 0.79 | 62 | 0.03 | 2.30 | 16.6 | 829 | 361 | 0.46 | 30 | 66 |
| Comparative Example 3 | 10 | EVOH coating | 160 | 0.25 | 640 | 0.05 | 1.03 | 2.0 | 100 | 97 | 0.46 | 162 | 489 |
| Comparative Example 4 | 11 | None | 155 | 0.26 | 596 | 0.12 | 0.53 | - | - | - | 0.26 | 132 | 98 |
| Comparative Example 5 | 12 | None | 144 | 0.21 | 686 | 0.17 | 0.31 | - | - | - | 0.40 | 126 | 96 |

The virus concentration rates in cases of Samples 1 to 7 were as follows. In the case of Sample 1, the virus concentration rate exceeded 900%. In the case of Sample 2, the virus concentration rate exceeded 600%. In the case of Sample 3, the virus concentration rate exceeded 300%. In the case of Sample 4, the virus concentration rate exceeded 400%. In the case of Sample 5, the virus concentration rate was 156%. In the case of Sample 6, the virus concentration rate exceeded 700%. In the case of Sample 7, the virus concentration rate exceeded 200%. From the above, in the concentration devices using the concentration membranes of Samples 1 to 7, a virus concentration rate exceeding at least 150% was observed, and thus the effect of concentrating the biological particles was remarkable.

On the other hand, the virus concentration rates in cases of Samples 8 to 12 were as follows. In the case of Sample 8, the virus concentration rate was 89%. In the case of Sample 9, the virus concentration rate was 66%. In the case of Sample 10, the virus concentration rate was 489%. In the case of Sample 11, the virus concentration rate was 98%. In the case of Sample 12, the virus concentration rate was 96%. From the above, the virus concentration rates in the concentration devices using the concentration membranes of Samples 8, 9, 11, and 12 were all less than 100%, and the effect of concentrating the biological particles was not observed at all.

The treatment times in the cases of Samples 1 to 7 were as follows. In the case of Sample 1, the treatment time was 40 seconds. In the case of Sample 2, the treatment time was 34 seconds. In the case of Sample 3, the treatment time was 72 seconds. In the case of Sample 4, the treatment time was 26 seconds. In the case of Sample 5, the treatment time was 60 seconds. In the case of Sample 6, the treatment time was 71 seconds. In the case of Sample 7, the treatment time was 96 seconds. From the above, the treatment times in the concentration devices using the concentration membranes of Samples 1 to 7 were 100 seconds or less, and concentration could be performed rapidly.

The treatment times in the cases of Samples 8 to 12 were as follows. In the case of Sample 8, the treatment time was 47 seconds. In the case of Sample 9, the treatment time was 30 seconds. In the case of Sample 10, the treatment time was 162 seconds. In the case of Sample 11, the treatment time was 132 seconds. In the case of Sample 12, the treatment time was 126 seconds. From the above, the treatment times in the concentration devices using the concentration membranes of Samples 10 to 12 exceeded 100 seconds, and concentration could not be performed rapidly.

From the above results, in all the concentration devices using the concentration membrane of Samples 1 to 7, the virus concentration rate exceeded 150% and the treatment time was 100 seconds or less, that is, both a high concentration rate of the biological particles and a rapid treatment time were achieved. Thus, they were considered to be practically useful.

On the other hand, in the concentration devices using the concentration membranes of Samples 8 to 12, the virus concentration rate was less than 150%, the treatment time exceeded 100 seconds, or both, that is, either or both of a high concentration rate of the biological particles and a rapid treatment time was/were missing. Thus, they were considered not to be suitable for practical use.

The disclosure of Japanese Patent Application No. 2019-047540 filed on March 14, 2019, is hereby incorporated by reference in their entirety. The disclosure of Japanese Patent Application No. 2019-047541 filed on March 14, 2019, is hereby incorporated by reference in their entirety.

All the documents, patent applications and technical standards that are described in the present specification are hereby incorporated by reference to the same extent as if each individual document, patent application or technical standard is concretely and individually described to be incorporated by reference.

Reference Numerals List

10: Concentration device
14: Piece to be folded and removed
20: Housing
21: Inlet
22: Outlet
23: Inner wall portion
24: Concentration space portion
25: Guide groove
30: Concentration membrane
33: Frame member
40: Liquid to be treated
41: Concentrated liquid
42: Effluent
50: Biological particle
60: Syringe

61:     Plunger
70:     Concentration system
71:     Pressurization unit
72:     Depressurization unit
80:     Waste liquid tank
81:     Exhaust port
82:     Suction port
90:     Guard unit

**Claims**

1.  A concentration membrane for use in concentrating biological particles, comprising:

    a hydrophilic composite porous membrane comprising:

    a porous substrate; and
    a hydrophilic resin with which at least one main surface and inner surfaces of pores of the porous substrate are coated,

    the hydrophilic composite porous membrane having a ratio t/x of a membrane thickness t ($\mu$m) to an average pore diameter x ($\mu$m), as measured with a perm porometer, of from 50 to 630.

2.  The concentration membrane according to claim 1, wherein the average pore diameter x of the hydrophilic composite porous membrane is from 0.1 $\mu$m to 0.5 $\mu$m.

3.  The concentration membrane according to claim 1 or 2, wherein the hydrophilic composite porous membrane has a bubble point pore diameter y of more than 0.8 $\mu$m and equal to or less than 3 $\mu$m, as measured with a perm porometer.

4.  The concentration membrane according to any one of claims 1 to 3, wherein the hydrophilic composite porous membrane has a ratio f/y of a water flow rate f (mL/(min · cm$^2$ · MPa)) to a bubble point pore diameter y ($\mu$m), as measured with a perm porometer, of from 100 to 480.

5.  The concentration membrane according to any one of claims 1 to 4, wherein the membrane thickness t of the hydrophilic composite porous membrane is from 10 $\mu$m to 150 $\mu$m.

6.  The concentration membrane according to any one of claims 1 to 5, wherein the hydrophilic composite porous membrane has a surface roughness Ra of from 0.3 $\mu$m to 0.7 $\mu$m.

7.  The concentration membrane according to any one of claims 1 to 6, wherein the hydrophilic resin comprises a hydrophilic resin in which a polymer main chain is composed only of a carbon atom and a side chain has at least one functional group selected from the group consisting of a hydroxy group, a carboxy group, and a sulfo group.

8.  The concentration membrane according to any one of claims 1 to 7, wherein the hydrophilic resin comprises at least one hydrophilic resin selected from the group consisting of polyvinyl alcohol, an olefin/vinyl alcohol-based resin, an acryl/vinyl alcohol-based resin, a methacryl/vinyl alcohol-based resin, a vinyl pyrrolidone/vinyl alcohol-based resin, polyacrylic acid, polymethacrylic acid, a perfluorosulfonic acid resin, and polystyrene sulfonic acid.

9.  The concentration membrane according to any one of claims 1 to 8, wherein the hydrophilic resin comprises an olefin/vinyl alcohol-based resin.

10. The concentration membrane according to any one of claims 1 to 9, wherein the porous substrate is a polyolefin microporous membrane.

11. The concentration membrane according to any one of claims 1 to 10, wherein the biological particles are 10 nm to 1,000 nm in size.

12. The concentration membrane according to any one of claims 1 to 11, wherein the biological particles are viruses, bacteria, or exosomes.

13. A concentration device for biological particles comprising:

a housing having an inlet and an outlet, wherein, due to a differential pressure between the inlet and the outlet, a liquid to be treated containing biological particles and water is injected from the inlet and discharged from the outlet;

a concentration membrane provided to separate the inlet and the outlet from each other in the housing, the concentration membrane being a hydrophilic porous membrane onto which the biological particles are not adsorbed, the concentration membrane allowing an effluent, which is a liquid having a concentration that is a concentration of the biological particles subtracted from a concentration of the liquid to be treated, to permeate from a surface on a side of the inlet to a surface on a side of the outlet; and

a concentration space portion that is a space on an upstream side of the concentration membrane in the housing and that stores a concentrated liquid which is a liquid having a concentration that is a concentration of the biological particles added to a concentration of the liquid to be treated by the concentration membrane.

14. The concentration device for biological particles according to claim 13, wherein, in the housing, a volume of the concentration space portion is from 0.05 cm$^3$ to 5 cm$^3$.

15. The concentration device for biological particles according to claim 13 or 14, wherein, in the housing, a filtration area of the concentration membrane is from 1 cm$^2$ to 20 cm$^2$.

16. The concentration device for biological particles according to any one of claims 13 to 15, wherein, in the housing, an inner wall portion facing the concentration space portion is formed with a guide groove continuous from the inlet.

17. The concentration device for biological particles according to any one of claims 13 to 16, wherein, in the housing, an inner wall portion facing the concentration space portion has a shape in which a diameter gradually increases from the inlet toward the concentration membrane.

18. The concentration device for biological particles according to any one of claims 13 to 17, wherein the concentration membrane comprises:
a hydrophilic composite porous membrane comprising:

a porous substrate, and
a hydrophilic resin with which at least one main surface and inner surfaces of pores of the porous substrate are coated.

19. The concentration device for biological particles according to claim 18, wherein the porous substrate is a polyolefin microporous membrane.

20. The concentration device for biological particles according to claim 18 or 19, wherein the hydrophilic resin comprises a hydrophilic resin in which a polymer main chain is composed only of a carbon atom and a side chain has at least one functional group selected from the group consisting of a hydroxy group, a carboxy group, and a sulfo group.

21. The concentration device for biological particles according to any one of claims 18 to 20, wherein the hydrophilic resin comprises at least one hydrophilic resin selected from the group consisting of polyvinyl alcohol, an olefin/vinyl alcohol-based resin, an acryl/vinyl alcohol-based resin, a methacryl/vinyl alcohol-based resin, a vinyl pyrrolidone/vinyl alcohol-based resin, polyacrylic acid, polymethacrylic acid, a perfluorosulfonic acid resin, and polystyrene sulfonic acid.

22. The concentration device for biological particles according to any one of claims 18 to 21, wherein the hydrophilic resin comprises an olefin/vinyl alcohol-based resin.

23. The concentration device for biological particles according to any one of claims 13 to 22, wherein the concentration membrane has a ratio t/x of a membrane thickness t ($\mu$m) to an average pore diameter x ($\mu$m), as measured with a perm porometer, of from 50 to 630.

**24.** The concentration device for biological particles according to any one of claims 13 to 23, wherein the membrane thickness t of the concentration membrane is from 10 $\mu$m to 150 $\mu$m.

**25.** The concentration device for biological particles according to any one of claims 13 to 24, wherein the average pore diameter x, as measured with a perm porometer, of the concentration membrane is from 0.1 $\mu$m to 0.5 $\mu$m.

**26.** The concentration device for biological particles according to any one of claims 13 to 25, wherein the concentration membrane has a bubble point pore diameter y of more than 0.8 $\mu$m and equal to or less than 3 $\mu$m, as measured with a perm porometer.

**27.** The concentration device for biological particles according to any one of claims 13 to 26, wherein the concentration membrane has a ratio f/y of a water flow rate f (mL/(min $\cdot$ cm$^2$ $\cdot$ MPa)) to a bubble point pore diameter y ($\mu$m), as measured with a perm porometer, of from 100 to 480.

**28.** The concentration device for biological particles according to any one of claims 13 to 27, wherein the concentration membrane has a surface roughness Ra of from 0.3 $\mu$m to 0.7 $\mu$m.

**29.** A concentration system for biological particles comprising:

the concentration device for biological particles according to any one of claims 13 to 28; and
a unit for applying a differential pressure between the inlet and the outlet.

**30.** A method for concentrating biological particles, comprising steps of:

supplying the liquid to be treated to the concentration device for biological particles according to any one of claims 13 to 28;
applying a differential pressure between the inlet and the outlet of the concentration device to obtain the concentrated liquid in the concentration space portion; and
recovering the concentrated liquid from the concentration space portion.

**31.** A method for detecting biological particles, comprising steps of:

supplying the liquid to be treated to the concentration device for biological particles according to any one of claims 13 to 28;
applying a differential pressure between the inlet and the outlet of the concentration device to obtain the concentrated liquid in the concentration space portion;
recovering the concentrated liquid from the concentration space portion; and
detecting the biological particles contained in the collected concentrated liquid.

FIG.1

10

21

20

22

FIG.2

10

24

21

23

20

22

30

## FIG.3

## FIG.4

EP 3 933 023 A1

FIG.5

FIG.6

FIG.7

FIG.8

FIG.9

10

21

20

22

FIG.10

10

21

20

22

FIG.11

FIG.12

FIG.13

FIG.14

FIG.15

FIG.16

FIG.17

FIG.18

## FIG.19

## FIG.20

## FIG.21

FIG.22

FIG.23

FIG.24

FIG.25

FIG.26

FIG.27

FIG.28

FIG.29

FIG.30

FIG.31

FIG.32

## FIG.33

FIG.34

FIG.35

FIG.36

FIG.37

## FIG.38

FIG.39

FIG.40

FIG.41

FIG.42

EP 3 933 023 A1

FIG.43

# FIG.44 (a)

PLUNGER

SYRINGE

VIRUS SUSPENSION

VIRUS

FILTER HOLDER

CONCENTRATION MEMBRANE
(HYDROPHILIC COMPOSITE POROUS
MEMBRANE)

FILTRATE

FIG.44 (b)

concentrated liquid

## INTERNATIONAL SEARCH REPORT

International application No.

PCT/JP2020/002968

### A. CLASSIFICATION OF SUBJECT MATTER

C12M 1/12(2006.01)i; B01D 61/14(2006.01)i; B01D 69/02(2006.01)i; B01D 69/12(2006.01)i; B01D 71/26(2006.01)i; B01D 71/38(2006.01)i; C12N 7/02(2006.01)i; C12Q 1/04(2006.01)i; G01N 33/48(2006.01)i
FI:      C12M1/12; B01D61/14 500; B01D69/02; B01D69/12; B01D71/26; B01D71/38; C12N7/02; C12Q1/04; G01N33/48 P

According to International Patent Classification (IPC) or to both national classification and IPC

### B. FIELDS SEARCHED

Minimum documentation searched (classification system followed by classification symbols)
C12M1/12; B01D61/14; B01D69/02; B01D69/12; B01D71/26; B01D71/38; C12N7/02; C12Q1/04; G01N33/48

Documentation searched other than minimum documentation to the extent that such documents are included in the fields searched
Published examined utility model applications of Japan      1922–1996
Published unexamined utility model applications of Japan      1971–2020
Registered utility model specifications of Japan      1996–2020
Published registered utility model applications of Japan      1994–2020

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2012/115021 A1 (DIC CORP.) 30.08.2012 (2012-08-30) claims, paragraphs [0021], [0034], examples | 1-31 |
| Y | | 1-31 |
| Y | JP 5-76733 A (KURABO INDUSTRIES LTD.) 30.03.1993 (1993-03-30) claims, paragraphs [0001], [0022], [0024] | 1-31 |

☐ Further documents are listed in the continuation of Box C.      ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 April 2020 (16.04.2020) | 28 April 2020 (28.04.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2020/002968

| Patent Documents referred in the Report | Publication Date | Patent Family | Publication Date |
|---|---|---|---|
| WO 2012/115021 A1 | 30 Aug. 2012 | (Family: none) | |
| JP 5-76733 A | 30 Mar. 1993 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11090184 A
- JP 2013531236 A
- JP 2009183804 A
- JP 2003268152 A
- JP H06057143 A
- JP 2004016930 A
- JP 2002265658 A
- JP 2016534748 A
- JP 2006071478 A
- JP S61271003 A
- JP 2019047540 A
- JP 2019047541 A